# FASCICULE DE BREVET EUROPEEN

(11) **EP 0 806 959 B1**
(45) Date de publication et mention de la délivrance du brevet: **13.11.2002**
(21) Numéro de dépôt: 96901848.0
(22) Date de dépôt: 24.01.1996
(51) Int. Cl.: A61K 35/14, C12N 5/08

(54) **PROCEDE DE PREPARATION DE MACROPHAGES ET TROUSSES POUR LA MISE EN OEUVRE DE CE PROCEDE**
VERFAHREN ZUR HERSTELLUNG VON MAKROPHAGEN UND AUSRÜSTUNGEN ZUR DURCHFÜHRUNG DIESES VERFAHRENS
METHOD FOR PREPARING MACROPHAGES AND KITS THEREFOR

(30) Priorité: 24.01.1995 FR 9500785
(43) Date de publication de la demande: 19.11.1997
(73) Titulaire: I.D.M. IMMUNO-DESIGNED MOLECULES, 75011 Paris (FR)
(72) Inventeur: ROMET-LEMONNE, Jean-Loup, F-75003 Paris (FR); CHOKRI, Mohamed, F-95170 Deuil-la-Barre (FR)
(74) Mandataire: Grosset-Fournier, Chantal Catherine
(86) Numéro de dépôt international: FR9600121
(87) Numéro de publication internationale: WO96022781

(56) Documents cités:
- WO-A-94/26875
- ANTICANCER RESEARCH, vol. 12, no. 6B, pages 2257-2260, CHOKRI M. ET AL. 'Production of Human Macrophages with Potent Antitumor Properties (MAK) by Culture of Monocytes in the Presence of GM-CSF and 1,25-Dihydroxy vitamin D3'
- INT J ARTIF ORGANS 14 (5). 1991. 304-312, FARADJI A ET AL 'APHERESIS- ELUTRIATION PROGRAM FOR ADOPTIVE IMMUNOTHERAPY WITH AUTOLOGOUS ACTIVATED MONOCYTES IN CANCER PATIENTS.'
- J IMMUNOL METHODS 159 (1-2). 1993. 29-38, LOPEZ M ET AL 'AUTOLOGOUS LYMPHOCYTES PREVENT THE DEATH OF MONOCYTES IN CULTURE AND PROMOTE AS DO GM-CSF IL-3 AND M-CSF THEIR DIFFERENTIATION INTO MACROPHAGES.'

## Description

La présente invention a pour objet un procédé de préparation de macrophages, et plus particulièrement de macrophages humains, notamment de macrophages activés (encore désignés macrophages cytotoxiques), ou encore de macrophages (ou autres cellules dérivées des monocytes ou de leurs précurseurs) pour la présentation d'antigènes, ainsi que des trousses et des compositions susceptibles d'être utilisées pour la mise en oeuvre de ce procédé.

Les macrophages jouent un rôle majeur dans la réponse antitumorale, ils peuvent être activés par des activateurs immunologiques contre des cellules cancéreuses (Adams D. and Hamilton T.: Activation of macrophages for tumor cell kill: effector mechanism and regulation. In Heppner & Fulton (eds), Macrophages and cancer. CRC Press, 1988, p. 27; Fidler I.: Macrophages and metastases. A biological approach to cancer therapy. Cancer Res, 45: 4714, 1985).

Par ailleurs, les macrophages, ou autres cellules dérivées des monocytes ou de leurs précurseurs, par leur forte capacité d'endocytose, de disgestion et de présentation à leur surface d'antigènes, sont capables d'induire une réponse immunitaire spécifique. A ce titre, ils représentent de bons candidats pour la préparation de vaccins, et plus particulièrement de vaccins cellulaires autologues. Les macrophages, ou autres cellules dérivées des monocytes, présentant à leur surface des antigènes particuliers capables d'induire une réponse immunitaire spécifique, sont encore désignés dans la suite de ce texte par l'expression MD-APC (pour "Monocytes Derived Antigen Presenting Cells").

Les MD-APC sont obtenues par culture de cellules mononucléées (monocytes ou leurs précurseurs) prélevées sur un patient ou sur sujet sain, en présence d'un (ou plusieurs) antigène(s) que l'on souhaite exprimer sous forme de fragments à la surface des cellules obtenues en fin de période de culture.

Les antigènes susceptibles d'être mis en culture avec les cellules mononucléées susmentionnées sont par exemple des antigènes exprimés par des cellules tumorales (notamment des fragments de cellules tumorales tuées) ou par des pathogènes (notamment des fragments de protéines de bactéries ou de virus).

Les MD-APC ainsi obtenues sont utilisées pour la préparation de vaccins dirigés contre la pathologie associée à l'antigène co-cultivé avec les cellules mononucléées (notamment des vaccins contre des maladies tumorales ou virales) et plus particulièrement pour la préparation de vaccins cellulaires autologues qui sont avantageusement administrés au patient chez lequel les cellules mononucléées de départ ont été prélevées.

Des MD-APC peuvent également être obtenues par transfection des cellules susmentionnées mises en culture, ou des cellules différenciées, notamment des macrophages, obtenues après culture, avec des séquences d'ADN codant pour des fragments d'antigènes tels que définis ci-dessus.

Des macrophages présentant une activité cytotoxique particulièrement importante (encore désignés macrophages activés ou MAK (marque déposée)) ainsi qu'un milieu de culture et un procédé d'obtention de tels macrophages ont fait l'objet de la demande internationale n° WO 9426875, déposée le 18 mai 1993.

Le procédé de préparation des macrophages défini dans la demande internationale susmentionnée, est réalisé à partir d'une composition enrichie en cellules sanguines obtenue par cytaphérèse réalisée sur un individu sain, et comprend une étape de culture de monocytes dans un milieu de culture contenant de la vitamine D3 1,25-dihydroxy et du GM-CSF (Granulocytes-Macrophages colony stimulating factor). Avantageusement, les monocytes sont cultivés en présence des lymphocytes, pendant environ 6 à 7 jours, et les macrophages différenciés ainsi obtenus sont activés par addition d'interféron γ (IFN-γ) dans le milieu de culture.

Cette étape de culture est, comme dans tout procédé d'obtention de macrophages décrit jusqu'à ce jour, précédée par une étape de séparation d'une part des cellules mononucléaires, et d'autre part des globules rouges, granulocytes et d'une partie des plaquettes contenus dans la composition dérivée du sang obtenue par cytaphérèse, et par une étape d'élimination, par lavage, d'une partie des plaquettes sanguines et des anticoagulants restant après l'étape précédente.

L'étape de séparation susmentionnée des globules rouges et granulocytes est généralement réalisée par centrifugation du milieu contenant les monocytes dans un gradient de densité, notamment dans une solution ayant une densité d'environ 1,0 à environ 1,3 g/ml, telle qu'une solution du type Ficoll Paque (Pharmacia) ayant une densité de 1,077 g/ml.

Dans la demande internationale susmentionnée, la composition dérivée du sang et obtenue par cytaphérèse contient environ 7 à 9.10⁹ leucocytes pour un volume d'environ 200 ml. Après l'étape de séparation des globules rouges et des granulocytes par centrifugation sur un gradient de densité, la composition de la suspension cellulaire mise en culture contient alors, pour un volume d'environ 500 ml à 1000 ml:

| | | |
|---|---|---|
| Hématocrite (Globules rouges) | < 0,1% | en nombre de globules blancs présents dans la composition |
| Plaquettes | < 10¹⁰ | |
| Globules blancs | 3 à 5.10⁹ | |
| Lymphocytes | 50-70% | en nombre de globules blancs présents dans la composition |
| Monocytes | 30-50% | en nombre de globules blancs présents dans la composition |
| Polynucléaires (ou granulocytes) | < 5% | en nombre de globules blancs présents dans la composition |

L'élimination des globules rouges et des granulocytes, préalablement à l'étape de culture, a toujours été considérée comme une étape indispensable en raison notamment du fait que ces derniers sont susceptibles d'inhiber totalement ou partiellement la différenciation des monocytes en macrophages lors de l'étape de culture.

Cette inhibition serait principalement due au fait que les globules rouges, les granulocytes, et les plaquettes utilisent les constituants du milieu de culture pour leur propre métabolisme, épuisant ainsi les réserves nécessaires au développement des monocytes, et entraînant une acidification du milieu de culture dans des proportions telles que l'étape de culture ne peut être amenée jusqu'à son terme (qui est d'environ 6 à 7 jours).

Par ailleurs, les granulocytes et les plaquettes seraient notamment susceptibles de sécréter des facteurs suppresseurs de la fonctionalité des macrophages, tels que le TGF (Transforming growth factor), et les prostaglandines.

Ainsi, il est couramment admis que les granulocytes ainsi que le plus de plaquettes possible doivent être séparés des monocytes avant la mise en culture de ces derniers, bien que cette étape de séparation entraîne également une élimination d'environ 20% à environ 50%, notamment d'environ 30% des cellules mononuclées présentes dans le milieu avant séparation.

La présente invention a pour but de fournir un nouveau procédé d'obtention de macrophages ou de MD-APC, le cas échéant activés, qui soit plus simple à mettre en oeuvre et présentant de meilleurs rendements que les procédés d'obtention des macrophages décrits jusqu'à maintenant.

Un autre but de l'invention est de fournir des trousses (ou kits) pour la mise en oeuvre de ce procédé.

Un autre but de la présente invention est de fournir à l'utilisateur des trousses contenant des compositions utilisables en tant que témoins internes pour le contrôle contrôle du suivi des différentes étapes du procédé, notamment pour le contrôle de la teneur en monocytes, et le cas échéant en autres constituants de la composition de départ du procédé de l'invention, et pour le contrôle de la teneur en macrophages ou de MD-APC, le cas échéant activés, de la composition finale recherchée.

La présente invention est illustrée à l'aide des figures suivantes:
- la figure 1 représente le montage pour la mise en oeuvre de la déplaquettisation et du lavage du produit de la cytaphérèse,
- la figure 2 représente le montage de la mise en culture des cellules mononucléées pour l'obtention de macrophages ou MD-APC,
- la figure 3 représente le montage du système d'élutriation.

La présente invention a pour objet un procédé d'obtention de macrophages ou de MD-APC, le cas échéant activés, ne comprenant pas d'étape de séparation par voie physique, notamment par centrifugation sur un gradient de densité de la manière indiquée ci-dessus, d'une part des globules rouges et/ou des granulocytes, et, d'autre part, des autres globules blancs, notamment des monocytes, présents dans la composition dérivée du sang et obtenue par cytaphérèse.

L'invention a plus particulièrement pour objet tout procédé de préparation d'une composition comprenant des macrophages ou des MD-APC, le cas échéant activés, caractérisé en ce que, partant d'une composition dérivée du sang d'origine humaine, et enrichie en cellules sanguines, et plus particulièrement en globules blancs tels que les monocytes, ou en précurseurs de ces derniers, notamment d'une composition dérivée du sang telle qu'obtenue par cytaphérèse, ledit procédé comprend les étapes suivantes:
- avantageusement une dilution de ladite composition dérivée du sang, notamment dans environ 2 à 3 fois le volume de cette dernière, à l'aide d'une solution physiologique appropriée,
- une étape de lavage de ladite composition dérivée du sang, avantageusement par centrifugation simple et lavage du culot issu de la centrifugation susmentionnée, après récupération du culot dans une solution physiologique de lavage appropriée, notamment dans une poche (du type poche de transfert), en effectuant par exemple une pression sur ladite poche, la solution de lavage étant alors éliminée vers une autre poche ou autre récipient, pour récupérer une composition dépourvue d'éventuels anticoagulants et de résidus divers et appauvrie en plaquettes,
- le cas échéant répétition de l'opération de lavage susmentionnée, notamment entre 1 et 2 fois,
- une étape de culture des cellules contenues dans la composition dérivée du sang obtenue après l'étape de lavage susmentionnée, en plaçant celle-ci dans un milieu de culture approprié, notamment dans une poche avantageusement hydrophobe, pendant environ 6 à environ 10 jours (notamment d'environ 6 à 7 jours), cette étape de culture étant le cas échéant effectuée en présence des antigènes particuliers susmentionnés, à savoir notamment en présence d'antigènes caractéristiques de cellules tumorales ou de pathogènes dans le cas de la production de MD-APC, et éventuellement suivie par une étape d'activation des macrophages ou MD-APC obtenus dans le milieu de culture, par addition dans ledit milieu de culture d'un activateur, tel que l'interféron γ, ledit activateur étant mis en contact avec le contenu du milieu de culture pendant environ 16 à environ 24 heures, cette étape de culture, et le cas échéant d'activation, étant éventuellement suivie par une centrifugation du milieu de culture et lavage du culot issu de la centrifugation, notamment de la manière indiquée ci-dessus,
cette étape de culture, et le cas échéant d'activation, étant:
. précédée par une étape d'élimination de tout ou partie des constituants autres que les monocytes, ou leurs précurseurs, susceptibles d'être présents dans la composition de départ, notamment des plaquettes, des globules rouges, des granulocytes et des lymphocytes, par mise en contact de la composition dérivée du sang obtenue après l'étape de lavage précédant l'étape de culture, avec des anticorps dirigés contre tout ou partie des constituants susmentionnés, et récupération de la solution contenant les monocytes, ou leurs précurseurs, tout ou partie des constituants susmentionnés restant fixés aux anticorps,
   et/ou suivie par une étape d'élimination de tout ou partie des constituants autres que les macrophages ou MD-APC par mise en contact de la composition dérivée du sang obtenue après l'étape de culture avec les anticorps tels que décrits ci-dessus, et récupération de la solution contenant les macrophages ou MD-APC, tout ou partie des constituants susmentionnés restant fixés aux anticorps,
. et/ou suivie par une étape de purification, notamment par élutriation, dans laquelle les macrophages ou les MD-APC, le cas échéant activés, sont séparés des autres constituants de la composition obtenue après l'étape de culture, et le cas échéant d'activation, susmentionnée, notamment des plaquettes, des globules rouges et des lymphocytes, par voie physique.

L'invention découle de la découverte faite par les inventeurs que, contrairement aux idées reçues dans ce domaine, il est possible d'amener la culture des monocytes, ou leurs précurseurs, en macrophages ou MD-APC à son terme avec de bons rendements lorsque le milieu de culture contient des quantités maîtrisées en globules rouges, lymphocytes, granulocytes et plaquettes.

L'invention offre donc l'avantage de disposer d'un procédé d'obtention de macrophages ou de MD-APC permettant de s'affranchir de l'étape de séparation par voie physique d'une part des cellules mononucléées, et plus particulièrement des monocytes, et d'autre part des globules rouges, des granulocytes (et de la grande majorité, sinon la totalité des plaquettes) contenus dans la composition dérivée du sang et obtenue par cytaphérèse, cette étape étant coûteuse (nécessitant la possession d'un matériel sophistiqué) et longue à mettre en oeuvre.

Avantageusement, la composition de départ dérivée du sang d'origine humaine dans le cadre de la mise en oeuvre du procédé susmentionné de l'invention, à savoir la composition de suspension cellulaire enrichie en cellules sanguines, telle qu'issue de la cytaphérèse, et avant l'étape de lavage précédent l'étape de culture dans le procédé décrit ci-dessus, est telle qu'elle comprend une proportion de monocytes supérieure à environ 5%, notamment d'environ 10% à environ 30%, en nombre de globules blancs présents dans ladite composition.

Une telle composition de départ, est avantageusement obtenue par mise en oeuvre d'un procédé de cytaphérèse réalisé sur un patient, ce procédé étant notamment effectué selon toute technique et avec tout matériel connus de l'homme de l'art dans ce domaine, le cas échéant, selon une procédure adaptée pour l'obtention d'une composition de départ telle que définie ci-dessus et ci-après.

Avantageusement, la composition de départ dérivée du sang d'origine humaine susmentionnée, peut contenir jusqu'à environ 3% de globules rouges, et de préférence est telle qu'elle comprend une proportion de globules rouges inférieure à environ 1%, notamment de 0,3% à 0,5%, en nombre de globules blancs présents dans ladite composition.

Avantageusement encore, la composition de départ dérivée du sang d'origine humaine susmentionnée, est telle que:
- le nombre de plaquettes est compris entre environ 2.10¹¹ à environ 6.10¹¹ pour un volume d'environ 150 ml à environ 200 ml,
- le nombre de globules blancs est compris entre environ 5.10⁹ à environ 5.10¹⁰ pour un volume d'environ 150 ml à environ 200 ml,
- la proportion de lymphocytes est comprise entre environ 60% à environ 80%, en nombre de globules blancs présents dans ladite composition,
- la proportion de monocytes est comprise entre environ 10% à environ 30%, en nombre de globules blancs présents dans ladite composition,
- la proportion de granulocytes est comprise entre environ 10% à environ 20% en nombre de globules blancs présents dans ladite composition.

L'étape de lavage de la composition de départ, de préférence répétée 2 fois, permet essentiellement d'éliminer une partie des plaquettes, et le cas échéant des réactifs utilisés lors de l'obtention de la composition de départ, notamment lors de la cytaphérèse, tels que les anticoagulants. Avantageusement, le pourcentage de plaquettes éliminées lors de cette étape est d'environ 80% à environ 90%.

La composition obtenue après l'étape de lavage de la composition de départ, contient avantageusement, dans un volume d'environ 600 ml, les constituants suivants:
- Hématocrite: 0,2 à 0,4% en nombre de globules blancs présents dans ladite composition
- Plaquettes: environ 2 à environ 6.10¹⁰
- Globules blancs: environ 4.10⁹ à environ 4.10¹⁰
- Lymphocytes: environ 60% à environ 80% en nombre de globules blancs présents dans ladite composition
- Monocytes: environ 10% à environ 20% en nombre de globules blancs présents dans ladite composition
- Polynucléaires: environ 10% à environ 15% en nombre de globules blancs présents dans ladite composition.

Selon un mode de réalisation préférée du procédé de l'invention, l'étape de culture et de différenciation des monocytes, ou de leurs précurseurs, en macrophages ou en MD-APC, est réalisée à partir d'une composition dérivée du sang d'origine humaine contenant, non seulement des cellules mononucléées, à savoir des monocytes ou leurs précurseurs, et des lymphocytes, mais aussi des globules rouges (dans des quantités appropriées, notamment dans les proportions indiquées ci-dessus), des granulocytes et des plaquettes, ladite étape de culture étant notamment réalisée à partir de la composition obtenue après l'étape de lavage de la composition de départ, telle que décrite ci-dessus.

S'agissant de l'étape de culture elle-même, celle-ci est de préférence effectuée pendant environ 6 jours à environ 10 jours dans un milieu de culture spécifique et prêt à l'emploi à base d'IMDM (Iscove Modified Dubelcco Medium, Gibco) modifié, à savoir le milieu de Dubelcco modifié par Iscove (IMDM) auquel on a ajouté de la L-Glutamine (2mM, Gibco) ou avantageusement de la L-Alanyl-L-Glutamine (2mM, Gibco), de l'acide pyruvique (2mM, Gibco), de l'Indométhacine (5.10⁻⁶ M, Sigma), du mercaptoéthanol (3.10⁻⁵ M, Gibco), et des acides aminés non essentiels (2%, Gibco). Au moment de la mise en culture, le milieu sera complété par 2 à 5 % de sérum AB⁺ (ou de sérum autologue) ainsi que des antibiotiques tels que la pénicilline et la streptomycine.

Le milieu de culture prêt à l'emploi susmentionné contient de la L-Alanyl-L-Glutamine en remplacement de la L-Glutamine. Les Inventeurs ont en effet mis en évidence que le milieu prêt à l'emploi susmentionné contenant de la L-Alanyl-L-Glutamine peut avantageusement être conservé à 4°C avec l'ensemble des constituants des kits de l'invention décrits ci-après, tandis que le milieu prêt à l'emploi susmentionné contenant de la L-Glutamine doit être conservé à -20°C.

Pour la différenciation des monocytes en macrophages, le milieu de culture susmentionné est avantageusement complété par du GMCSF et de la vitamine D3.

Pour la différenciation des monocytes, ou de leurs précurseurs, en MD-APC, le milieu de culture susmentionné est utilisé pour la co-culture avec les antigènes décrits ci-dessus, et est avantageusement complété par une à plusieurs cytokines telles que le GMCSF, le TNF-α, l'IL-13, l'IL-4, et/ou par d'autres adjuvants tels que le calcitriol et l'histamine.

Dans le cas de l'obtention de macrophages ou de MD-APC activés, un activateur, tel que l'interféron γ, est avantageusement introduit dans le milieu de culture susmentionné environ 16 heures à environ 24 heures avant l'arrêt de la culture.

La composition de la suspension cellulaire obtenue après l'étape de culture, dans le cadre de la mise en oeuvre du procédé préféré décrit ci-dessus, dans un volume d'environ 600 ml à 2000 ml, est avantageusement la suivante:
- Lymphocytes : environ 2.10⁹ à environ 2.10¹⁰
- Macrophages (ou MD-APC): environ 6.10⁸ à environ 6.10⁹

Cette dernière composition comprend peu ou pas de granulocytes, ces derniers s'étant en grande partie décomposés durant l'étape de culture.

Les rendements de l'étape de culture susmentionnée, sont avantageusement d'environ 80% en cellules mononucléées, et d'environ 50% à environ 70% pour la différenciation en macrophages ou MD-APC (en prenant en compte le nombre de monocytes de départ).

Les rendements susmentionnés sont calculés en faisant le rapport entre le nombre de macrophages ou MD-APC obtenus après l'étape de culture et le nombre de monocytes présents dans la composition avant culture.

Avantageusement, dans le mode de réalisation préférée du procédé de l'invention décrit ci-dessus, l'étape de culture, et le cas échéant d'activation, susmentionnée est suivie par une étape de purification notamment par élutriation, dans laquelle les macrophages ou les MD-APC, le cas échéant activés, sont séparés des autres constituants de la composition obtenue après l'étape de culture, et le cas échéant d'activation, susmentionnée, notamment des plaquettes, des globules rouges et des lymphocytes, par voie physique.

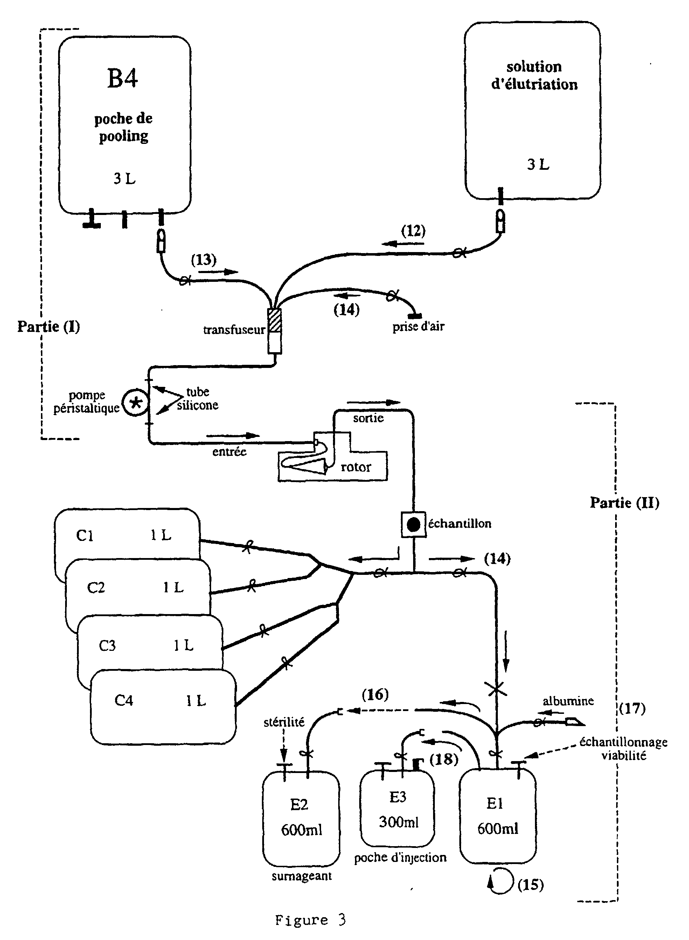

Avantageusement, l'étape de culture, et le cas échéant d'activation, mise en oeuvre dans le cadre du procédé préféré susmentionné, est suivie d'une étape d'élutriation telle que représentée sur la figure 3, cette dernière étant de préférence réalisée en utilisant le dispositif suivant:
- une poche (désignée B4 sur la figure 3) contenant la composition comprenant les macrophages ou les MD-APC, le cas échéant activés, obtenue après l'étape de culture, et le cas échéant d'activation, ainsi qu'une poche contenant une solution physiologique (encore désignée solution d'élutriation) susceptible de maintenir l'ensemble du système d'élutriation dans un milieu viable pour les macrophages ou MD-APC, le cas echéant activés, et une prise d'air, sont reliées à un transfuseur à trois branches muni d'un filtre ,
- la prise d'air étant fermée, une pompe péristaltique entraîne le contenu de la poche B4 dans un élutriateur équipé d'un rotor avec une chambre d'élutriation, l'alimentation de l'ensemble du circuit d'élutriation en liquide physiologique étant assurée par l'entraînement du contenu de la poche contenant la solution d'élutriation, à l'aide de la pompe susmentionnée, pendant toute la durée de l'élutriation,
- la vitesse de rotation du rotor de l'élutriateur et le débit de la pompe sont tels que les divers constituants de la composition de la poche B4, introduits dans le rotor, sont séparés en fonction de leur taille dans la chambre d'élutriation, les constituants de plus petite taille migrant en premier vers le haut de la chambre (proche de la sortie de l'élutriateur), tandis que les constituants de plus grande taille, à savoir les macrophages ou MD-APC, le cas échéant activés, migrent vers la sortie de la chambre en dernier,
- les différents constituants de la composition contenue dans la poche B4, ainsi séparés sont récoltés séparément dans des poches reliées à l'élutriateur, les plaquettes et divers résidus de petite taille étant récupérés les premiers dans une poche C1, les globules rouges étant ensuite récupérés dans une poche C2, puis les lymphocytes dans les poches C3 et C4, et enfin les macrophages ou MD-APC dans une poche E1, les différentes catégories susmentionnées de constituants étant extraites de l'élutriateur, en fonction de leur taille respective, soit en diminuant la vitesse de rotation de l'élutriateur, soit en augmentant le débit de la pompe, la récupération des macrophages ou MD-APC dans la poche E1 pouvant avantageusement être réalisée par arrêt du rotor de l'élutriateur et ouverture de la prise d'air, la poche E1 contenant ainsi la composition recherchée de macrophages ou MD-APC, le cas échéant activés, avantageusement sous forme essentiellement pure.

En variante du procédé préféré décrit ci-dessus, l'étape de culture et différenciation des monocytes ou de leurs précurseurs en macrophages ou MD-APC, peut être précédée par une étape d'élimination de tout ou partie des constituants autres que les cellules mononucléées, en particulier autres que les monocytes, susceptibles d'être contenus dans la composition obtenue après l'étape de lavage de la composition de départ, telle que décrite ci-dessus, ladite étape d'élimination étant effectuée non pas par voie physique, mais par mise en oeuvre de réactions immunologiques entre tout ou partie des constituants susmentionnés et des anticorps susceptibles de reconnaître lesdits constituants.

L'étape d'élimination par anticorps susmentionnée, est avantageusement réalisée par mise en contact de la composition issue de l'étape de lavage de la composition de départ, avec des anticorps anti-plaquettes, et/ou anti-globules rouges et/ou anti-granulocytes fixés sur un support solide, notamment sur les parois d'une poche du type transfert, ou sur les parois de tout autre dispositif dans lequel ladite composition issue de l'étape initiale de lavage est susceptible d'être introduite, ledit dispositif étant le cas échéant aménagé de manière à autoriser la circulation de ladite composition le long des parois sur lesquelles sont fixés lesdits anticorps.

La mise en contact de ladite composition avec lesdits anticorps est réalisée, soit simultanément avec tout ou partie des anticorps susmentionnés, soit successivement notamment par introduction de ladite composition dans une poche ou autre dispositif tel que décrit ci-dessus, contenant des anticorps anti-plaquettes, puis passage de ladite composition dans une seconde poche contenant des anticorps anti-globules rouges, et/ou passage de ladite composition dans une troisième poche contenant des anticorps anti-granulocytes, les lymphocytes étant éliminés lors de l'étape d'élutriation suivant l'étape de culture.

Selon un autre mode de réalisation préférée du procédé de l'invention, ce dernier ne comprend pas d'étape d'élutriation, et l'étape de culture, et le cas échéant d'activation, est:
- précédée par une étape de mise en contact de la composition issue de l'étape de lavage de la composition de départ, avec des anticorps anti-plaquettes, et/ou anti-globules rouges et/ou anti-granulocytes fixés sur un support solide, notamment de la manière indiquée ci-dessus,
   la mise en contact de ladite composition avec lesdits anticorps étant réalisée, soit simultanément avec tout ou partie des anticorps susmentionnés, soit successivement notamment de la manière indiquée ci-dessus,
   ladite composition étant ensuite transférée dans la poche contenant le milieu de culture,
- et suivie par une étape de mise en contact de la composition issue de l'étape de culture, et le cas échéant d'activation, avec des anticorps anti-lymphocytes, par introduction de ladite composition dans une (ou plusieurs) poche(s) ou autre dispositif tel que décrit ci-dessus, contenant des anticorps anti-lymphocytes, et récupération de la composition recherchée de macrophages ou MD-APC, le cas échéant activés, sous forme essentiellement pure.

Avantageusement, lorsque le procédé préféré décrit ci-dessus est réalisé par mise en contact de la composition issue de l'étape de lavage de la composition de départ, successivement avec des anticorps anti-globules rouges, puis anti-plaquettes, puis anti-granulocytes, la composition de la suspension cellulaire ainsi obtenue avant l'étape de culture, dans un volume d'environ 600 ml à environ 2000 ml, est avantageusement la suivante:
- Globules rouges: < 0,1%
- Plaquettes: < 10¹⁰
- Globules blancs: environ 3.10⁹ à environ 3.10¹⁰
- Lymphocytes: environ 60% à environ 80% en nombre de globules blancs présents dans ladite composition
- Monocytes: environ 20% à environ 30% en nombre de globules blancs présents dans ladite composition
- Polynucléaires: < 5%.

La composition de la suspension cellulaire obtenue après l'étape de culture, dans le cadre de la mise en oeuvre du procédé préféré décrit ci-dessus, dans un volume d'environ 600 ml à 2000 ml, est avantageusement identique à celle de la suspension cellulaire obtenue après l'étape de culture dans le cadre de la mise en oeuvre du procédé préféré précédent ne faisant pas appel à une étape de réaction immunologique préalablement à l'étape de culture, à savoir:
- Lymphocytes: environ 2.10⁹ à environ 2.10¹⁰
- Macrophages: environ 6.10⁸ à environ 6.10⁹

Les rendements de l'étape de culture susmentionnée, sont avantageusement identiques à ceux décrits dans le cadre du procédé préféré précédent, à savoir environ 80% en cellules mononucléées, et environ 50% à environ 70% pour la différenciation en macrophages (en prenant en compte le nombre de monocytes de départ).

Les rendements des procédés décrits ci-dessus, sont avantageusement du même ordre, que ces procédés soient réalisés suivant le principe de l'élutriation après l'étape de culture, ou suivant le principe de la réaction immunologique avant et après l'étape de culture.

Ainsi, les procédés décrits ci-dessus de l'invention sont tels que l'on obtient des suspensions cellulaires comprenant des macrophages ou MD-APC, le cas échéant activés, sous forme essentiellement pure, notamment dans des proportions d'environ 80% à environ 95%, et avec des rendements finals d'environ 20% à environ 50% (lesdits rendements étant calculés en faisant le rapport entre la quantité de macrophages ou MD-APC purifiés obtenus en final et la quantité de monocytes mis en culture au départ).

Selon un mode de réalisation particulièrement avantageux des procédés de l'invention décrits ci-dessus, la composition de la suspension cellulaire de départ, et/ou l'une au moins des compositions de suspension cellulaire obtenue aux différentes étapes des procédés susmentionnés, et/ou la composition de la suspension cellulaire finale, peu(ven)t être comparées à des compositions dites témoins internes, en vue du suivi du bon déroulement des procédés en question.

Un témoin interne particulièrement avantageux est celui constitué d'une quantité déterminée de monocytes, purifiés à partir du sang prélevé chez un individu sain, avantageusement fixés, notamment par lyophilisation, ledit témoin interne permettant de quantifier le nombre de monocytes présents dans la composition de départ.

Un témoin interne particulièrement préféré est tel qu'il comprend environ 2.10⁶ à environ 4.10⁶ monocytes lyophilisés pour un volume d'environ 1 ml à environ 2 ml d'une solution aqueuse.

La quantification du nombre de monocytes présents dans la composition de départ, est avantageusement réalisée par prélèvement d'une partie aliquote de la composition de départ, marquage des monocytes susceptibles d'être présents dans la fraction prélevée, notamment à l'aide d'anticorps marqués, par exemple de façon radioactive, enzymatique ou par fluorescence, révélation du marquage effectué à l'aide de tout dispositif approprié, et comparaison à l'intensité de marquage obtenue dans les mêmes conditions opératoires avec ledit témoin interne.

Les anticorps marqués susceptibles de reconnaître des antigènes de surface spécifiques des monocytes sont avantageusement ceux dirigés contre par exemple les antigènes suivants: CD14, HLADR et le CD64.

Un autre témoin interne particulièrement avantageux est celui constitué d'une quantité déterminée de macrophages ou MD-APC purifiés, et le cas échéant activés, à partir du sang prélevé chez un individu sain, avantageusement fixés, notamment par lyophilisation, puis reconstitués dans une solution adéquate de 1 à 2 ml, ledit témoin interne permettant de quantifier le nombre de macrophages ou de MD-APC, le cas échéant activés, présents dans la composition finale.

Un témoin interne particulièrement préféré est tel qu'il comprend environ 2.10⁶ à environ 4.10⁶ macrophages lyophilisés pour un volume d'environ 1 ml à environ 2 ml d'une solution aqueuse.

La quantification du nombre de macrophages ou MD-APC présents dans la composition finale, est avantageusement réalisée par prélèvement d'une partie aliquote de la composition finale, marquage des macrophages susceptibles d'être présents dans la fraction prélevée, notamment à l'aide d'anticorps marqués, par exemple de façon radioactive, enzymatique ou par fluorescence, révélation du marquage effectué à l'aide de tout dispositif approprié, et comparaison à l'intensité de marquage obtenue dans les mêmes conditions opératoires avec ledit témoin interne.

Les anticorps marqués susceptibles de reconnaître des antigènes de surface spécifiques des macrophages et/ou des MD-APC sont avantageusement dirigés contre l'un au moins des antigènes suivants: CD54, CD58, CD80, CD86, CD14, CD16, MAX-1, CD64, HLADR décrits notamment dans les articles suivants: Andreesen et al., Biology **47**: 490-497 (1990), Lopez et al., Journal of Immunotherapy **11**: 209-217 (1992), Chokri et al., Anticancer Research **12**: 2257-2260 (1992).

Avantageusement, les témoins internes susmentionnés sont constitués de billes, choisies notamment parmi les billes fluorescentes, présentant un diamètre comparable à celui des monocytes ou macrophages, et susceptibles de donner des signaux identiques à ceux donnés par les monocytes et les macrophages dans les solutions de départ et finale respectivement.

Selon un autre mode de réalisation particulièrement avantageux des procédés décrits ci-dessus de l'invention, le contrôle des compositions de macrophages ou de MD-APC, le cas échéant activés, obtenues lors de la mise en oeuvre de ces procédés, peut être effectuée par détection, et le cas échéant mesure, des cytokines sécrétées par les macrophages dans lesdites compositions, et comparaison au profil standard de sécrétion de cytokines par les macrophages ou MD-APC, le cas échéant activés.

A titre d'illustration, la détection des cytokines sécrétées, comme l'IL-1 (Interleukine 1), l'IL-6 et le TNF-α (Tumor Necrosis Factor-α), peut être effectuée sur un prélèvement d'une partie aliquote du milieu de culture dans lequel la différenciation et l'activation des macrophages obtenus ont eu lieu.

Les macrophages et les MD-APC, le cas échéant activés, obtenus par le procédé selon l'invention, peuvent être testés pour leur capacité à reconnaître et tuer des cellules tumorales (notamment par un test de cytotoxicité), ou, dans le cas des MD-APC, pour leur capacité de présentation antigènique, notamment en vérifiant leur capacité à induire une lymphoprolifération allogènique (par exemple test MLR, Mixed Lymphocyte Reaction). Ces tests permettront de déterminer le nombre d'unité fonctionnelle en macrophages ou MD-APC obtenu par le procédé de l'invention.

L'invention a également pour objet des trousses (ou kits) pour l'obtention de compositions de macrophages ou de MD-APC, le cas échéant activés, caractérisée en ce qu'elles comprennent:
. les éléments nécessaires aux lavages, la mise en culture et l'élutriation, comprenant:
   - une (ou plusieurs) poche(s) de lavage,
   - une (ou plusieurs) poche(s) de culture, de préférence hydrophobe(s),
   - un (ou plusieurs) connecteur(s) pour l'élimination du surnageant des lavages,
   - une (ou plusieurs) tubulure(s) de connexion entre les poches précédentes,
   - le cas échéant, un (ou plusieurs) site(s) d'injection dans la (ou les) poche(s) de lavage et de culture,
   - un (ou plusieurs) système(s) permettant l'ouverture ou la fermeture des liaisons entre la (ou les) poche(s) de lavage et la (ou les) poche(s) de culture, notamment des systèmes de clamp sur les tubulures,
   - deux poches contenant la solution physiologique pour les lavages et l'élutriation,
   - un transfuseur trois branches avec prise d'air,
   - une tubulure en silicone et un raccord au rotor servant de liaison entre le transfuseur et le rotor de l'élutriateur,
   - une (ou plusieurs) poche(s) de collecte, le cas échéant reliées entre elles par des tubulures munies d'un système de clamp,
   - une tubulure et un raccord au rotor servant de liaison entre la (ou les) poche(s) de collecte susmentionnée(s) et le rotor de l'élutriateur,
. les réactifs nécessaires à la mise en culture, la différenciation, l'activation et le contrôle, comprenant:
   - une poche de milieu de culture spécifique et prêt à l'emploi, notamment un milieu de culture à base d'IMDM modifié, à savoir un milieu Iscove modified Dubelcco medium (IMDM, Gibco), auquel on a ajouté de la L-Glutamine (2 mM, Gibco) ou de la L-Alanyl-L-Glutamine (2 mM, Gibco), de l'acide pyruvique (2 mM, Gibco), de l'Indométhacine (5.10⁻⁶ M, Sigma), du mercaptoéthanol (3.10⁻⁵ M, Gibco), des acides aminés non essentiels (2%, Gibco) ; 2 à 5% de sérum AB⁺ (ou de sérum autologue), ainsi que des antibiotiques tels que pénicilline et streptomycine, sont ajoutés au moment de la culture,
   - un ou plusieurs suppléments avantageusement sous forme liquide ou lyophilisée, à additionner au milieu de culture, choisis parmi les suivants:
      * du GMCSF,
      * de la vitamine D3,
      * de l'interleukine-13 (IL-13),
      * de l'IL-4
      * du TNF-α
      * de l'histamine,
   - le cas échéant un activateur tel que l'interféron γ, avantageusement sous forme liquide ou lyophilisée,
   - avantageusement un (ou plusieurs) témoin(s) interne(s) comprenant des monocytes et/ou macrophages ou MD-APC, ces derniers étant le cas échéant activés, avantageusement fixés, ou lyophilisés, et le cas échéant marqués, ou des billes calibrées, le cas échéant marquées, notamment par fluorescence,
   - un ou plusieurs anticorps marqués, notamment par fluorescence, tels que ceux dirigés contre les antigènes CD3 (marqueur des lymphocytes utilisé en tant que témoin négatif), CD45 (marqueur des leucocytes en général), CD14, CD16, CD64, CD54, CD58, CD80, CD86, HLA-DR et MAX-1.

Une trousse particulièrement avantageuse dans le cadre de la présente invention, se présente sous forme de deux ensembles:
- l'ensemble I contenant les poches, tubulures, sites d'injection, systèmes de clamp, le transfuseur trois branches, à savoir les éléments dits "secs" utilisés pour les lavages, la mise en culture et la purification par élutriation,
- l'ensemble II contenant une poche contenant le milieu de culture spécifique ainsi que les réactifs pour la mise en culture, la différenciation, l'activation et le contrôle de qualité.

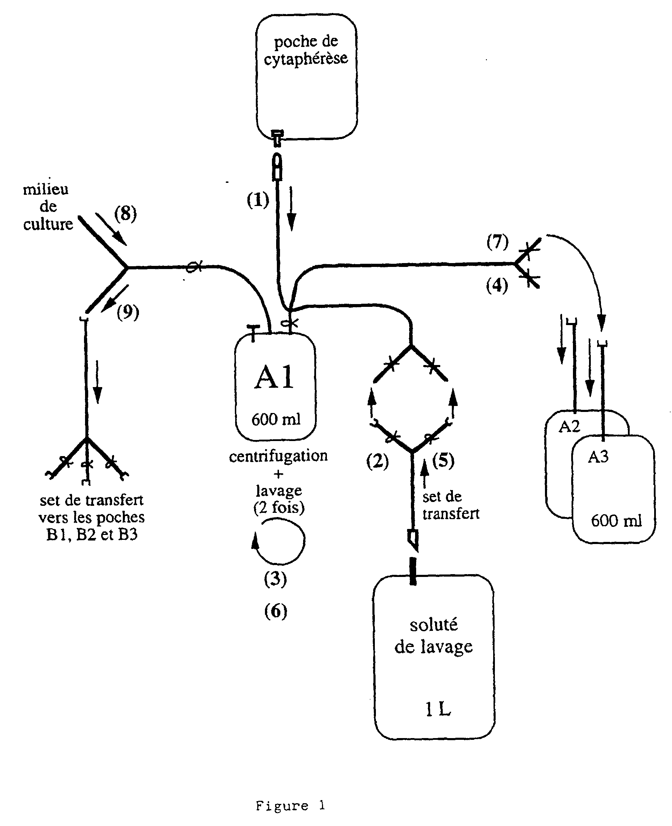

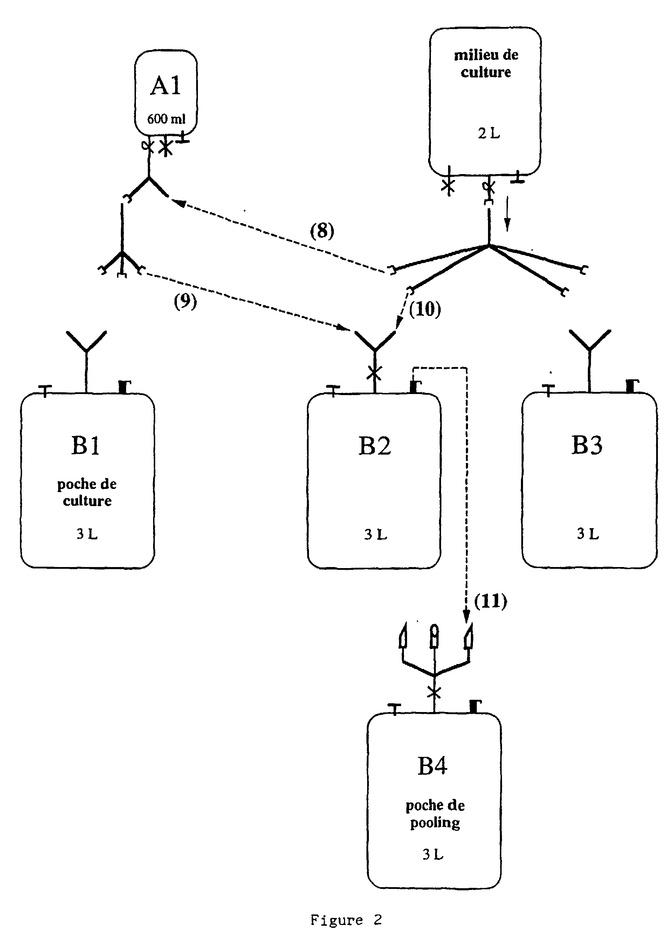

La trousse susmentionnée est davantage caractérisée en ce que:
. l'ensemble I est constitué de deux sous-ensembles sous forme de deux boîtes:
   - la boîte A contenant le matériel nécessaire pour les lavages des cellules provenant de la cytaphérèse, notamment:
      * trois poches d'une capacité de 600 ml (poche centrale A1, et poches de recueil A2 et A3 sur figure 1)
      * un set de transfert pour transférer le soluté de lavage dans la poche (poche A1 sur figure 1) dans laquelle est transférée la composition sanguine en vue de diluer et laver les cellules issues de la cytaphérèse,
      * un set de transfert des cellules lavées (contenues dans la poche A1 sur la figure 1) vers les poches de culture (poches B1, B2, et B3 sur figure 2),
      * un set de transfert du milieu de culture, dan un premier temps vers les cellules lavées (contenues dans la poche A1 sur la figure 1), et dans un deuxième temps vers les poches de culture susmentionnées,
      * trois poches de culture hydrophobes d'une capacité de 3 litres (poches B1, B2, et B3 sur figure 2),
   - la boîte B contenant les éléments pour séparer les macrophages ou MD-APC différenciés des autres cellules présentes dans la culture lors de l'étape d'élutriation, notamment:
      * une poche de regroupement (ou poche de pooling B4 sur figure 2)
      * un transfuseur trois branches avec prise d'air,
      * une tubulure en silicone, et deux raccords au rotor, la tubulure et l'un des deux raccords servant de liaison entre le transfuseur et le rotor de l'élutriateur, l'autre raccord servant de liaison avec ledit rotor et l'ensemble de poches ci-après, cette dernière tubulure contenant un site de prélèvement d'échantillon,
      * un ensemble composé de quatre poches de collecte (poches C1, C2, C3, et C4 sur figure 3) d'une capacité d'un litre, d'une poche (poche E1, sur la figure 3) d'une capacité 600 ml pour contenir la fraction finale des macrophages ou MD-APC purifiés, d'une poche (poche E2 sur figure 3) d'une capacité de 600 ml pour la récupération du surnageant de la poche précédente, et d'une poche (poche E3 sur la figure 3) pour l'injection aux patients des macrophages ou MD-APC, le cas échéant activés,
. l'ensemble II est représenté par la boîte C, cette dernière étant une boîte isotherme, le cas échéant avec refroidisseur pour maintenir la température à 4°C, et contenant notamment:
   - une poche de deux litres de milieu de culture à base d'IMDM modifié tel que décrit ci-dessus,
   - une boîte contenant les produits liquides ou lyophilisés suivants:
      * un flacon d'antibiotiques (pénicilline, avantageusement 1 000 U/streptomycine, avantageusement 1000 µg),
      * un flacon de vitamine D3 (avantageusement 4 à 8 µg),
      * un flacon de GMCSF (avantageusement 5.10⁵ à 10⁶ U),
      * un flacon d'interféron γ (avantageusement 25.10⁴ à 5.10⁵ U),
      * un flacon d'IL-13,
      * un flacon d'IL-4,
      * un flacon de TNFα,
      * un flacon d'histamine,
   - une boîte contenant un (ou plusieurs) témoin(s) interne(s) tels que décrits ci-dessus, et un ou plusieurs flacons contenant des anticorps anti-CD3, anti-CD45, anti-CD14, anti-CD16, anti-CD64, anti-MAX-1, anti-CD54, anti-CD58, anti-CD80, anti-CD86 et anti-HLA-DR marqués, notamment couplés à la fluorescéine.

L'invention vise également l'utilisation d'une trousse telle que définie ci-dessus, pour la mise en oeuvre d'un procédé tel que décrit ci-dessus selon l'invention comprenant une étape d'élutriation.

L'invention a également pour objet une trousse pour l'obtention de compositions de macrophages ou MD-APC, le cas échéant activés, caractérisée en ce qu'elle comprend:
- une (ou plusieurs) poche(s) à l'intérieur de laquelle (desquelles) se trouvent fixés des anticorps anti-globules rouges,
- une (ou plusieurs) poche(s) à l'intérieur de laquelle (desquelles) se trouvent fixés des anticorps anti-plaquettes,
- une (ou plusieurs) poche(s) à l'intérieur de laquelle (desquelles) se trouvent fixés des anticorps anti-granulocytes,
- une (ou plusieurs) poche(s) à l'intérieur de laquelle (desquelles) se trouvent fixés des anticorps anti-lymphocytes,
- une (ou plusieurs) poche(s) de culture,
- une (ou plusieurs) poche(s) de récupération des macrophages ou MD-APC, le cas échéant activés, purifiés,
- des tubulures de raccord entre les différentes poches susmentionnées,
- des suppléments avantageusement sous forme liquide ou lyophilisée, à additionner au milieu de culture, notamment:
   * des antibiotiques tels que pénicilline et streptomycine,
   * du GMCSF,
   * de la vitamine D3,
   * de l'IL-13,
   * de l'IL-4,
   * du TNFα,
   * de l'histamine,
- le cas échéant un activateur tel que l'interféron γ, avantageusement sous forme liquide ou lyophilisée,
- avantageusement un (ou plusieurs) témoin(s) interne(s) comprenant des monocytes ou macrophages ou MD-APC, le cas échéant activés, avantageusement fixés, et le cas échéant marqués, ou des billes calibrées, le cas échéant marquées, notamment par fluorescence, tel(s) que décrit(s) ci-dessus,
- des anticorps marqués, notamment par fluorescence, tels que ceux dirigés contre les antigènes CD3, CD45, CD14, CD16, CD64, CD54, CD58, CD80, CD86, MAX-1 et HLA-DR.

L'invention a plus particulièrement pour objet l'utilisation d'une trousse telle que décrite ci-dessus, pour la mise en oeuvre d'un procédé tel que décrit ci-dessus selon l'invention comprenant une étape de réaction immunologique.

L'invention concerne également toute composition liquide contenant une suspension cellulaire dérivée de sang d'origine humaine, caractérisée en ce qu'elle comprend une proportion de monocytes supérieure à environ 5%, notamment d'environ 10% à environ 30%, en nombre de globules blancs présents dans ladite composition.

L'invention a également pour objet toute composition telle que décrite ci-dessus, caractérisée en ce qu'elle comprend une proportion de globules rouges inférieure à environ 3%, ou inférieure à environ 1%, notamment de 0,3% à 0,5%, en nombre de globules blancs présents dans ladite composition.

L'invention a plus particulièrement pour objet toute composition telle que décrite ci-dessus, caractérisée en ce que:
- le nombre de plaquettes est compris entre environ 2.10¹¹ à environ 6.10¹¹ pour un volume d'environ 150 ml à environ 200 ml,
- le nombre de globules blancs est compris entre environ 5.10⁹ à environ 5.10¹⁰ pour un volume d'environ 150 ml à environ 200 ml,
- la proportion de lymphocytes est comprise entre environ 60% à environ 80%, en nombre de globules blancs présents dans ladite composition,
- la proportion de granulocytes est comprise entre environ 10% à environ 20% en nombre de globules blancs présents dans ladite composition.

Les compositions décrites ci-dessus sont susceptibles d'être utilisées en tant que témoins internes dans le cadre de la mise en oeuvre d'un procédé selon l'invention.

A titre d'exemple, le contrôle interne dans le cadre de la préparation de macrophages activés, se présente de la manière suivante;
Conditionnement :
- 1 flacon de lyophilisat (3x10⁶/flacon)
- 1 flacon de tampon de reconstitution
Reconstitution :
Réhydrater le flacon de lyophilisat avec 1 ml de la solution de reconstitution.
Mélanger doucement par retournement. Attendre au minimum 10 minutes avant l'utilisation de la préparation.
Utilisation :
- le volume du contrôle interne utilisé est de 100µl (3x10⁵ cellules)
- ajouter l'anticorps à tester
- incuber pendant 30 minutes à température ambiante
- ajouter 1 ml de PBS (ne pas laver)
- analyser au cytofluoromètre

Le pourcentage et l'intensité de marquage de macrophages ou MD-APC preparés seront comparés aux cellules produites.

Le tableau 1 qui suit donne une indication sur l'évolution des marqueurs membranaires en fonction du temps de stockage, des macrophages lyophilisés utilisés en tant que témoins internes. Les résultats sont exprimés en pourcentage de cellules positives et intensité de marquage. La ligne "Ctrl" correspond aux résultats obtenus avec des macrophages frais, non lyophilisés, cultivés et obtenus selon le procédé de l'invention. La ligne "7 Jours" correspond à des macrophages obtenus selon le procédé de l'invention, lyophilisés et réhydratés 7 jours après la lyophilisation. La ligne "7 Mois" correspond à des macrophages obtenus selon le procédé de l'invention, lyophilisés et réhydratés 7 mois après la lyophilisation. On constate que globalement les pourcentages de cellules positives ainsi que les intensités de marquage sont conservés entre les macrophages frais et ceux conservés à l'état lyophilisé pendant 7 jours ou 7 mois. Ainsi l'utilisateur des kits selon l'invention, est à même de pouvoir comparer les compositions qu'il obtient avec les témoins (ou standards) internes fournis dans ces kits, ces témoins étant avantageusement produits selon la même procédure et étant à l'état lyophilisé.

**Tableau 1**

| Marqueurs | | | | | | |
|---|---|---|---|---|---|---|
| | CD45 | CD3 | CD14 | CD16 | CD64 | HLADR |
| Ctrl | 92/115 | 0,3/54 | 87/149 | 22/35 | 77/63 | 84/115 |
| 7 jours | 95/131 | 3/30 | 96/119 | 10/30 | 93/59 | 96/169 |
| 7 mois | 91/119 | 3/22 | 86/95 | 10/25 | 81/54 | 82/116 |

Avantageusement les trousses selon l'invention sont accompagnées d'instructions écrites pour la préparation de macrophages ou MD-APC, le cas échéant activés.

Avantageusement, les trousses selon l'invention sont accompagnées également d'instructions sur support informatique permettant de guider l'opérateur pour la validation de chaque étape du procédé de l'invention, le but final étant de déterminer le nombre d'unités fonctionnelles susmentionnées obtenues par la mise en oeuvre du procédé de l'invention.

A titre d'illustration, les instructions écrites pour l'étape d'activation des macrophages, accompagnant un kit selon l'invention, peuvent se présenter de la manière suivante :

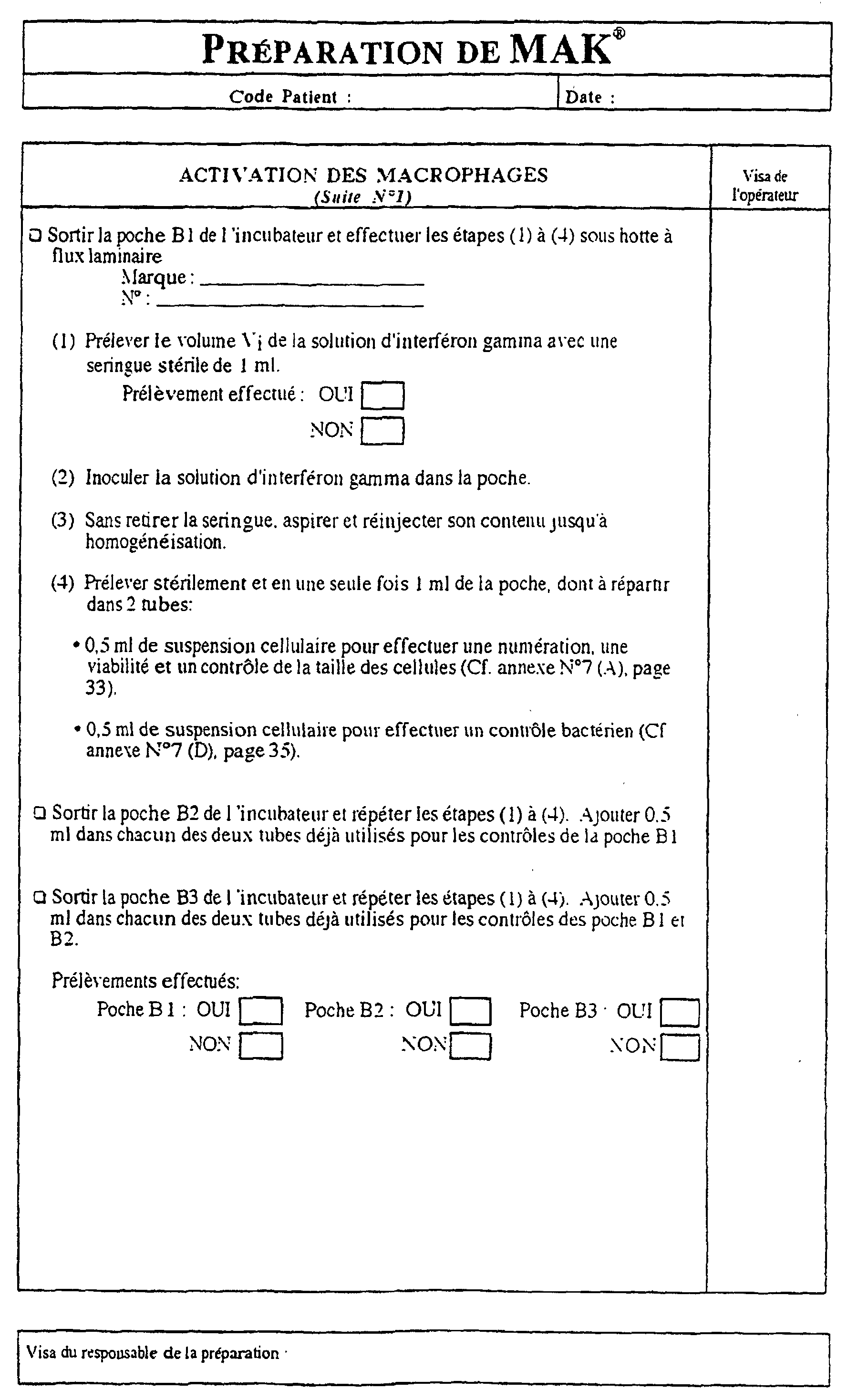

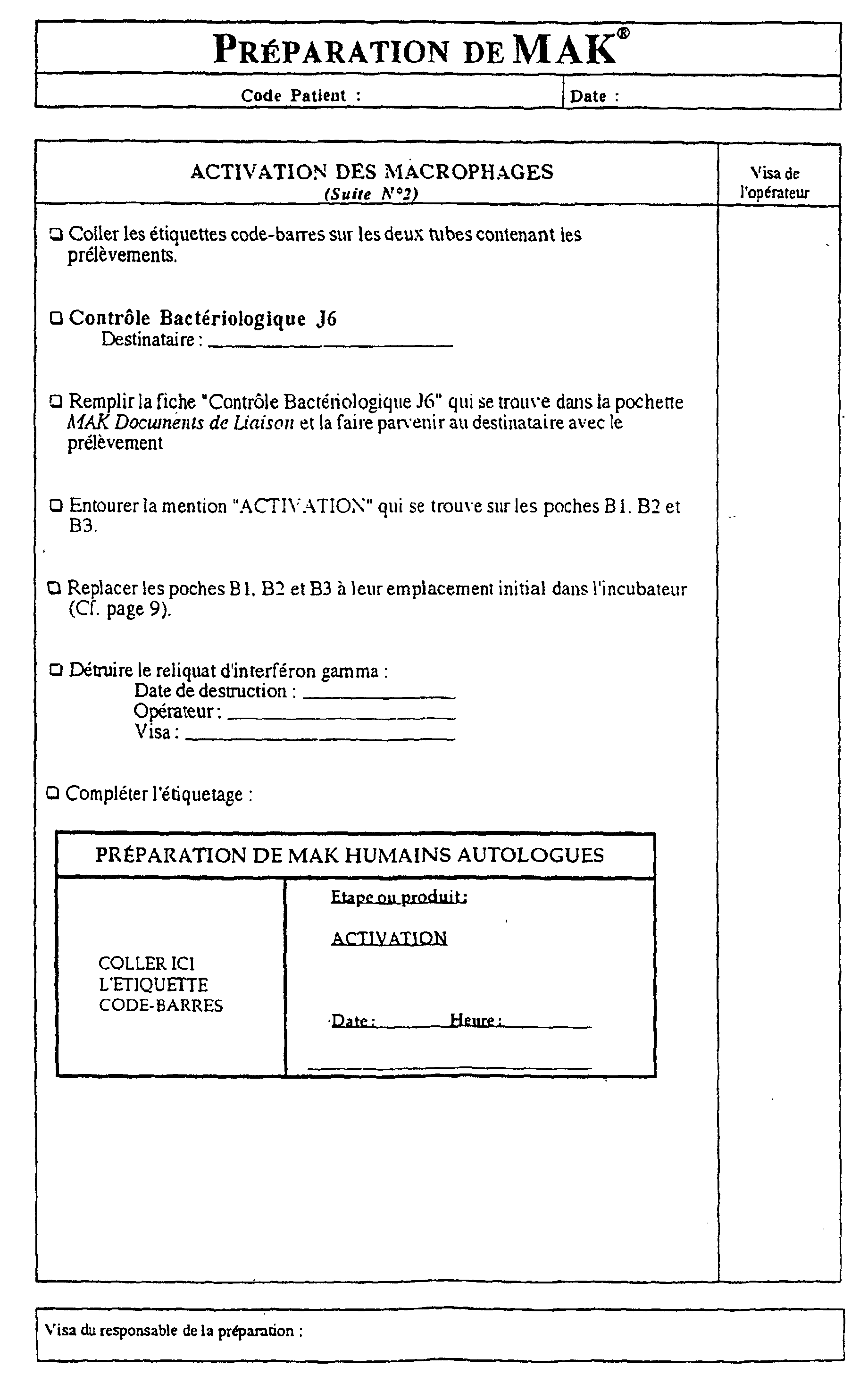

### Description des figures :

La description des opérations représentées sur les figures 1 à 3 est la suivante :

### I-DEPLAQUETTISATION DU PRODUIT DE CYTAPHERESE (figure 1)

(1) Transférer le contenu de la poche de cytaphérèse dans une poche A1 de 600 ml.
(2) Ajouter dans la poche contenant les cellules mononucléées un poids de solution saline tamponnée, tel que le poids final de la poche soit de 450-500 g.
(3) Centrifuger la poche A1 à 300 g, pendant 10 minutes à +4°C.
(4) Evacuer le surnageant dans une poche A2, à l'aide d'une presse.
(5) Compléter à 450 g par la solution saline.
(6) Centrifuger une deuxième fois la poche A1.
(7) Evacuer le surnageant dans la poche A3
   o Détacher les cellules de la poche de centrifugation A1, par friction
(8) Compléter à 600 ml avec le milieu de culture préalablement préparé
   o Noter le volume de milieu de culture ajouté
   o Prélever stérilement les échantillons de contrôle

### II-MISE EN CULTURE DES CELLULES MONONUCLEEES (figure 2)

(9) Répartir équitablement dans les poches de culture B1, B2 et B3
(10) Compléter le volume requis pour chaque poche par du milieu de culture.
(11) Transvaser le contenu des trois poches de culture dans la poche de pooling B4, après la fin de la période de culture et d'activation.

### III-SEPARATION PAR ELUTRIATION (figure 3)

o Monter sur l'entrée de la chambre d'élutriation stérilisée, la partie I (Kit d'entrée)
et sur la sortie de la chambre d'élutriation le kit de sortie (partie II)
(12) Remplir le circuit avec au moins 300 ml de solution d'élutriation.
(13) Simultanément, fermer l'arrivée de la solution d'élutriation et ouvrir celle de la poche de suspension cellulaire (poche B4) sans changer le débit de la pompe.

Dès que la totalité des cellules est passée, fermer l'arrivée de la poche des cellules B4 et ouvrir celle de la solution d'élutriation.

Augmenter le débit de la pompe tous les 500 ml et prélever un échantillon,
(14) Quand les cellules de taille macrophagique commencent à sortir, arrêter la centrifugation et dévier le prélèvement sur la poche de collecte E1 en fermant l'arrivée de la solution d'élutriation et en ouvrant la prise d'air.
(15) Centrifuger la poche E1 à 400 g pendant 10 min.
(16) Evacuer le surnageant dans la poche E2
(17) Remettre les cellules en suspension dans une solution de 250 ml d'albumine humaine à 4%. Bien homogénéiser
(18) Transvaser dans la poche d'injection E3.

La légende des figures 1 à 3 est la suivante :
→ représente le sens de circulation du fluide.
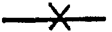 représente la soudure.
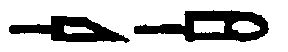 symbolise les perforateurs.
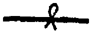 représente le clamp.
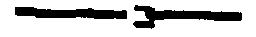 représente la connection.
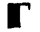 représente le site de connection.

## Revendications

1. Procédé de préparation d'une composition comprenant des macrophages, notamment des macrophages activés, ou des macrophages, ou autres cellules dérivées des monocytes, ou des précurseurs de ces derniers, présentant à leur surface des antigènes particuliers capables d'induire une réponse immunitaire spécifique (encore désignés MD-APC), ces MD-APC étant le cas échéant activés, **caractérisé en ce que**, partant d'une composition dérivée du sang d'origine humaine, et enrichie en cellules sanguines, et plus particulièrement en globules blancs tels que les monocytes, ou en précurseurs de ces derniers, notamment d'une composition dérivée du sang telle qu'obtenue par cytaphérèse, ledit procédé comprend les étapes suivantes:
- une étape de lavage de ladite composition dérivée du sang, avantageusement par centrifugation simple et lavage du culot issu de la centrifugation susmentionnée, après récupération du culot dans une solution physiologique de lavage appropriée, notamment dans une poche (du type poche de transfert), en effectuant par exemple une pression sur ladite poche, la solution de lavage étant alors éliminée vers une autre poche ou autre récipient, pour récupérer une composition dépourvue d'éventuels anticoagulants et de résidus divers et appauvrie en plaquettes,
- une étape de culture des cellules contenues dans la composition dérivée du sang obtenue après l'étape de lavage susmentionnée, en plaçant celle-ci dans un milieu de culture approprié, notamment dans une poche avantageusement hydrophobe, pendant environ 6 à environ 10 jours (notamment d'environ 6 à 7 jours), cette étape de culture étant le cas échéant effectuée en présence des antigènes particuliers susmentionnés, à savoir notamment en présence d'antigènes caractéristiques de cellules tumorales ou de pathogènes dans le cas de la production de MD-APC, et éventuellement suivie par une étape d'activation des macrophages ou MD-APC obtenus dans le milieu de culture, par addition dans ledit milieu de culture d'un activateur, tel que l'interféron γ, ledit activateur étant mis en contact avec le contenu du milieu de culture pendant environ 16 à environ 24 heures, cette étape de culture, et le cas échéant d'activation, étant éventuellement suivie par une centrifugation du milieu de culture et lavage du culot issu de la centrifugation, notamment de la manière indiquée ci-dessus,
cette étape de culture, et le cas échéant d'activation, étant:
. précédée par une étape d'élimination de tout ou partie des constituants autres que les monocytes, ou leurs précurseurs, susceptibles d'être présents dans la composition de départ, notamment des plaquettes, des globules rouges, des granulocytes et des lymphocytes, par mise en contact de la composition dérivée du sang obtenue après l'étape de lavage précédant l'étape de culture, avec des anticorps dirigés contre tout ou partie des constituants susmentionnés, et récupération de la solution contenant les monocytes, ou leurs précurseurs, tout ou partie des constituants susmentionnés restant fixés aux anticorps,
. et/ou suivie par une étape d'élimination de tout ou partie des constituants autres que les macrophages ou MD-APC par mise en contact de la composition dérivée du sang obtenue après l'étape de culture avec les anticorps tels que décrits ci-dessus, et récupération de la solution contenant les macrophages ou MD-APC, tout ou partie des constituants susmentionnés restant fixés aux anticorps,
. et/ou suivie par une étape de purification, notamment par élutriation, dans laquelle les macrophages ou les MD-APC, le cas échéant activés, sont séparés des autres constituants de la composition obtenue après l'étape de culture, et le cas échéant d'activation, susmentionnée, notamment des plaquettes, des globules rouges et des lymphocytes, par voie physique, sous réserve, avant la culture de cellules, qu'il n'y ait pas d'étape de séparation par voie physique d'une part des monocytes et d'autre part, des globules rouges, des granulocytes et de la grande majorité des plaquettes.

2. Procédé selon la revendication 1 comprenant une dilution de ladite composition dérivée du sang notamment dans environ 2 à 3 fois le volume de cette dernière, à l'aide d'une solution physiologique appropriée.

3. Procédé selon la revendication 1, **caractérisé en ce que** la composition de départ dérivée du sang d'origine humaine, est telle qu'elle comprend une proportion de monocytes supérieure à environ 5%, notamment d'environ 10% à environ 30%, en nombre de globules blancs présents dans ladite composition.

4. Procédé selon la revendication 1, **caractérisé en ce que** la composition de départ dérivée du sang d'origine humaine, est telle qu'elle comprend une proportion de globules rouges inférieure à environ 3%, notamment de 0,3% à 0,5%, en nombre de globules blancs présents dans ladite composition.

5. Procédé selon l'une des revendications 1 à **4**, **caractérisé en ce que** la composition de départ dérivée du sang d'origine humaine, est telle que:
- le nombre de plaquettes est compris entre environ 2.10¹¹ à environ 6.10¹¹ par 150 ml à 200 ml,
- le nombre de globules blancs est compris entre environ 5.10⁹ à environ 5.10¹⁰ par 150 ml à 200 ml,
- la proportion de lymphocytes est comprise entre environ 60% à environ 80%, en nombre de globules blancs présents dans ladite composition,
- la proportion de granulocytes est comprise entre environ 10% à environ 20% en nombre de globules blancs présents dans ladite composition.

6. Procédé selon l'une des revendications 1 à **5, caractérisé en ce que** les étapes de culture, d'activation et de centrifugation sont suivies d'une étape d'élutriation, et réalisée en utilisant le dispositif suivant:
- une poche contenant la composition comprenant les macrophages ou les MD-APC, le cas échéant activés, obtenue après l'étape de culture, et le cas échéant d'activation, ainsi qu'une poche contenant une solution physiologique (encore désignée solution d'élutriation) susceptible de maintenir l'ensemble du système d'élutriation dans un milieu viable pour les macrophages ou MD-APC, le cas echéant activés, et une prise d'air, sont reliées à un transfuseur à trois branches muni d'un filtre ,
- la prise d'air étant fermée, une pompe péristaltique entraîne le contenu de la poche dans un élutriateur équipé d'un rotor avec une chambre d'élutriation, l'alimentation de l'ensemble du circuit d'élutriation en liquide physiologique étant assurée par l'entraînement du contenu de la poche contenant la solution d'élutriation, à l'aide de la pompe susmentionnée, pendant toute la durée de l'élutriation,
- la vitesse de rotation du rotor de l'élutriateur et le débit de la pompe sont tels que les divers constituants de la composition de la poche, introduits dans le rotor, sont séparés en fonction de leur taille dans la chambre d'élutriation, les constituants de plus petite taille migrant en premier vers le haut de la chambre (proche de la sortie de l'élutriateur), tandis que les constituants de plus grande taille, à savoir les macrophages ou MD-APC, le cas échéant activés, migrent vers la sortie de la chambre en dernier,
- les différents constituants de la composition contenue dans la poche, ainsi séparés sont récoltés séparément dans des poches reliées à l'élutriateur, les plaquettes et divers résidus de petite taille étant récupérés les premiers dans une première poche, les globules rouges étant ensuite récupérés dans une deuxième poche, puis les lymphocytes dans les troisième et quatrième poches, et enfin les macrophages ou MD-APC dans une cinquième poche, les différentes catégories susmentionnées de constituants étant extraites de l'élutriateur, en fonction de leur taille respective, soit en diminuant la vitesse de rotation de l'élutriateur, soit en augmentant le débit de la pompe, la récupération des macrophages ou MD-APC dans la cinquième poche pouvant avantageusement être réalisée par arrêt du rotor de l'élutriateur et ouverture de la prise d'air, la cinquième poche contenant ainsi la composition recherchée de macrophages ou MD-APC, le cas échéant activés, avantageusement sous forme essentiellement pure.

7. Procédé selon l'une des revendications 1 à 5, **caractérisé en ce que** l'étape de culture, et le cas échéant d'activation, est:
- précédée par une étape de mise en contact de la composition issue de l'étape de lavage de la composition de départ, avec des anticorps anti-plaquettes, et/ou anti-globules rouges et/ou anti-granulocytes fixés sur un support solide, notamment de la manière indiquée ci-dessus,
la mise en contact de ladite composition avec lesdits anticorps étant réalisée, soit simultanément avec tout ou partie des anticorps susmentionnés, soit successivement notamment de la manière indiquée ci-dessus,
ladite composition étant ensuite transférée dans la poche contenant le milieu de culture,
- et suivie par une étape de mise en contact de la composition issue de l'étape de culture, et le cas échéant d'activation, avec des anticorps anti-lymphocytes, par introduction de ladite composition dans une (ou plusieurs) poche(s) ou autre dispositif tel que décrit ci-dessus, contenant des anticorps anti-lymphocytes, et récupération de la composition recherchée de macrophages ou MD-APC, le cas échéant activés, sous forme essentiellement pure.

8. Utilisation d'une trousse (ou kit) pour l'obtention de compositions de macrophages ou de MD-APC, le cas échéant activés, selon l'une des revendications 1 à 7 **caractérisée en ce qu'**elle comprend:
. les éléments nécessaires aux lavages, la mise en culture et l'élutriation, comprenant:
- une (ou plusieurs) poche(s) de lavage,
- une (ou plusieurs) poche(s) de culture, de préférence hydrophobe(s),
- un (ou plusieurs) connecteur(s) pour l'élimination du surnageant des lavages,
- une (ou plusieurs) tubulure(s) de connexion entre les poches précédentes,
- le cas échéant, un (ou plusieurs) site(s) d'injection dans la (ou les) poche(s) de lavage et de culture,
- un (ou plusieurs) système(s) permettant l'ouverture ou la fermeture des liaisons entre la (ou les) poche(s) de lavage et la (ou les) poche(s) de culture, notamment des systèmes de clamp sur les tubulures,
- deux poches contenant la solution physiologique pour les lavages et l'élutriation,
- un transfuseur trois branches avec prise d'air,
- une tubulure en silicone et un raccord au rotor servant de liaison entre le transfuseur et le rotor de l'élutriateur,
- une (ou plusieurs) poche(s) de collecte, le cas échéant reliées entre elles par des tubulures munies d'un système de clamp,
- une tubulure et un raccord au rotor servant de liaison entre la (ou les) poche(s) de collecte susmentionnée(s) et le rotor de l'élutriateur,
. les réactifs nécessaires à la mise en culture, la différenciation, l'activation et le contrôle, comprenant:
- une poche de milieu de culture spécifique et prêt à l'emploi, notamment un milieu de culture à base d'IMDM modifié, à savoir un milieu Iscove modified Dubelcco medium (IMDM, Gibco), auquel on a ajouté de la L-Glutamine (2 mM, Gibco) ou de la L-Alanyl-L-Glutamine (2 mM, Gibco), de l'acide pyruvique (2 mM, Gibco), de l'Indométhacine (5.10⁻⁶ M, Sigma), du mercaptoéthanol (3.10⁻⁵ M, Gibco), des acides aminés non essentiels (2%, Gibco) ; 2 à 5% de sérum AB⁺ (ou de sérum autologue), ainsi que des antibiotiques tels que pénicilline et streptomycine, sont ajoutés au moment de la culture,
- un ou plusieurs suppléments avantageusement sous forme liquide ou lyophilisée, à additionner au milieu de culture, choisis parmi les suivants:
* du GMCSF,
* de la vitamine D3,
* de l'interleukine-13 (IL-13),
* de l'IL-4
* du TNF-α
* de l'histamine,
- le cas échéant un activateur tel que l'interféron γ, avantageusement sous forme liquide ou lyophilisée,
- avantageusement un (ou plusieurs) témoin(s) interne(s) comprenant des monocytes et/ou macrophages ou MD-APC, ces derniers étant le cas échéant activés, avantageusement fixés, ou lyophilisés, et le cas échéant marqués, ou des billes calibrées, le cas échéant marquées, notamment par fluorescence,
- un ou plusieurs anticorps marqués, notamment par fluorescence, tels que ceux dirigés contre les antigènes CD3 (marqueur des lymphocytes utilisé en tant que témoin négatif), CD45 (marqueur des leucocytes en général), CD14, CD16, CD64, CD54, CD58, CD80, CD86, HLA-DR et MAX-1.

9. Utilisation selon la revendication **8**, **caractérisée en ce que** la trousse se présente sous forme de deux ensembles:
- l'ensemble I contenant les poches, tubulures, sites d'injection, systèmes de clamp, le transfuseur trois branches, à savoir les éléments dits "secs" utilisés pour les lavages, la mise en culture et la purification par élutriation,
- l'ensemble II contenant une poche contenant le milieu de culture spécifique ainsi que les réactifs pour la mise en culture, la différenciation, l'activation et le contrôle de qualité.

10. Utilisation selon la revendication **9, caractérisée en ce que**:
. l'ensemble I est constitué de deux sous-ensembles sous forme de deux boîtes:
- la boîte A contenant le matériel nécessaire pour les lavages des cellules provenant de la cytaphérèse, notamment:
* trois poches d'une capacité de 600 ml (poche centrale A1, et poches de recueil A2 et A3 sur figure 1)
* un set de transfert pour transférer le soluté de lavage dans la poche (poche A1 sur figure 1) dans laquelle est transférée la composition sanguine en vue de diluer et laver les cellules issues de la cytaphérèse,
* un set de transfert des cellules lavées (contenues dans la poche A1 sur la figure 1) vers les poches de culture (poches B1, B2, et B3 sur figure 2),
* un set de transfert du milieu de culture, dan un premier temps vers les cellules lavées (contenues dans la poche A1 sur la figure 1), et dans un deuxième temps vers les poches de culture susmentionnées,
* trois poches de culture hydrophobes d'une capacité de 3 litres (poches B1, B2, et B3 sur figure 2),
- la boîte B contenant les éléments pour séparer les macrophages ou MD-APC différenciés des autres cellules présentes dans la culture lors de l'étape d'élutriation, notamment:
* une poche de regroupement (ou poche de pooling B4 sur figure 2)
* un transfuseur trois branches avec prise d'air,
* une tubulure en silicone, et deux raccords au rotor, la tubulure et l'un des deux raccords servant de liaison entre le transfuseur et le rotor de l'élutriateur, l'autre raccord servant de liaison avec ledit rotor et l'ensemble de poches ci-après, cette dernière tubulure contenant un site de prélèvement d'échantillon,
* un ensemble composé de quatre poches de collecte (poches C1, C2, C3, et C4 sur figure 3) d'une capacité d'un litre, d'une poche (poche E1, sur la figure 3) d'une capacité 600 ml pour contenir la fraction finale des macrophages ou MD-APC purifiés, d'une poche (poche E2 sur figure 3) d'une capacité de 600 ml pour la récupération du surnageant de la poche précédente, et d'une poche (poche E3 sur la figure 3) pour l'injection aux patients des macrophages ou MD-APC, le cas échéant activés,
. l'ensemble II est représenté par la boîte C, cette dernière étant une boîte isotherme, le cas échéant avec refroidisseur pour maintenir la température à 4°C, et contenant notamment:
- une poche de deux litres de milieu de culture à base d'IMDM modifié tel que décrit ci-dessus,
- une boîte contenant les produits liquides ou lyophilisés suivants:
* un flacon d'antibiotiques (pénicilline, avantageusement 1 000 U/streptomycine, avantageusement 1000 µg),
* un flacon de vitamine D3 (avantageusement 4 à 8 µg),
* un flacon de GMCSF (avantageusement 5.10⁵ à 10⁶ U),
* un flacon d'interféron γ (avantageusement 25.10⁴ à 5.10⁵ U),
* un flacon d'IL-13,
* un flacon d'IL-4,
* un flacon de TNFα,
* un flacon d'histamine,
- une boîte contenant un (ou plusieurs) témoin(s) interne(s) tels que décrits ci-dessus, et un ou plusieurs flacons contenant des anticorps anti-CD3, anti-CD45, anti-CD14, anti-CD16, anti-CD64, anti-MAX-1, anti-CD54, anti-CD58, anti-CD80, anti-CD86 et anti-HLA-DR marqués, notamment couplés à la fluorescéine.

11. Trousse pour l'obtention de compositions de macrophages ou de MD-APC, le cas échéant activés, **caractérisée en ce qu'**elle comprend:
- une (ou plusieurs) poche(s) à l'intérieur de laquelle (desquelles) se trouvent fixés des anticorps anti-globules rouges,
- une (ou plusieurs) poche(s) à l'intérieur de laquelle (desquelles) se trouvent fixés des anticorps anti-plaquettes,
- une (ou plusieurs) poche(s) à l'intérieur de laquelle (desquelles) se trouvent fixés des anticorps anti-granulocytes,
- une (ou plusieurs) poche(s) à l'intérieur de laquelle (desquelles) se trouvent fixés des anticorps anti-lymphocytes,
- une (ou plusieurs) poche(s) de culture,
- une (ou plusieurs) poche(s) de récupération des macrophages ou MD-APC, le cas échéant activés, purifiés,
- des tubulures de raccord entre les différentes poches susmentionnées,
- des suppléments avantageusement sous forme liquide ou lyophilisée, à additionner au milieu de culture, notamment:
* des antibiotiques tels que pénicilline et streptomycine,
* du GMCSF,
* de la vitamine D3,
* de l'IL-13,
* de l'IL-4,
* du TNFα,
* de l'histamine,
- le cas échéant un activateur tel que l'interféron γ, avantageusement sous forme liquide ou lyophilisée,
- avantageusement un (ou plusieurs) témoin(s) inteme(s) comprenant des monocytes ou macrophages ou MD-APC, le cas échéant activés, avantageusement fixés, et le cas échéant marqués, ou des billes calibrées, le cas échéant marquées, notamment par fluorescence, tel(s) que décrit(s) ci-dessus,
- des anticorps marqués, notamment par fluorescence, tels que ceux dirigés contre les antigènes CD3, CD45, CD14, CD16, CD64, CD54, CD58, CD80, CD86, MAX-1 et HLA-DR.

12. Utilisation d'une trousse selon la revendication **12** pour la mise en oeuvre d'un procédé selon l'une des revendications 1 à **5** et **7**.

## Patentansprüche

1. Verfahren zum Herstellen einer Zusammensetzung, die umfasst: Makrophagen, insbesondere aktivierte Makrophagen, oder Makrophagen, oder andere von Monozyten abgeleitete Zellen, oder Vorstufen von letzteren, die an ihrer Oberfläche besondere Antigene aufweisen, die eine spezifische Immunantwort einführen (nachstehend als MD-APC bezeichnet), wobei diese MD-APC gegebenenfalls aktiviert sind, **dadurch gekennzeichnet, dass** ausgehend von einer Zusammensetzung, die von Blut menschlichen Ursprungs abgeleitet ist, und mit Blutzellen angereichert ist, und insbesondere mit weißen Blutkörperchen, wie etwa die Monozyten, oder mit Vorstufen von den letzteren, insbesondere eine von Blut abgeleitete Zusammensetzung, wie etwa durch Zytapherese erhalten, wobei das Verfahren die folgenden Stufen umfasst:
- einen Schritt des Waschens der von Blut abgeleiteten Zusammensetzung, vorzugsweise durch einfache Zentrifugation und Waschen des Bodensatzes, der aus der zuvor erwähnten Zentrifugation stammt, nach Wiedergewinnung des Bodensatzes in einer geeigneten physiologischen Waschlösung, insbesondere in einer Tasche (des Typs einer Transfertasche), wobei zum Beispiel ein Druck auf die Tasche ausgeübt wird, wobei die Waschlösung folglich in eine andere Tasche oder eine andere Empfangseinrichtung ausgeleert wird, um eine Zusammensetzung ohne eventuelle Antikoagulantien und diverse Rückstände und abgereichert an Blutplättchen zu erhalten,
- einen Schritt der Kultur von Zellen, die in der von Blut abgeleiteten Zusammensetzung enthalten sind, die nach dem zuvor erwähnten Waschschritt erhalten wurde, wobei diese in ein geeignetes Kulturmilieu zwischen ungefähr 6 bis ungefähr 10 Tagen, (insbesondere ungefähr 6 bis 7 Tage) platziert wird, insbesondere in einer vorzugsweise hydrophoben Tasche, wobei der Kulturschritt gegebenenfalls in Gegenwart von besonderen vorstehend erwähnten Antigenen ausgeführt, d. h. insbesondere in Gegenwart von charakteristischen Antigenen von Tumorzellen oder Keimen im Fall der Herstellung von MD-APC, und eventuell gefolgt von einem Schritt der Aktivierung der Makrophagen oder MD-APC, die im Kulturmilieu erhalten wurden, durch Zugabe eines Aktivators in das Kulturmilieu, wie etwas das Interferon-γ, wobei der Aktivator mit dem Inhalt des Kulturmilieus während ungefähr 16 bis ungefähr 24 Stunden in Kontakt gehalten wird, wobei auf den Schritt der Kultur, und gegebenenfalls der Aktivierung eine Zentrifugation des Milieus der Kultur und Waschen des aus der Zentrifugation stammenden Bodensatzes folgt, insbesondere auf die vorstehend angegebene Weise,
wobei dem Schritt der Kultur, und gegebenenfalls der Aktivierung:
. ein Schritt der Eliminierung von allen oder einen Teil der Bestandteile vorausgeht, die sich von Monozyten, oder deren Vorstufen unterscheiden, wie sie in der Zusammensetzung von Anfang an enthalten sind, insbesondere Blutplättchen, rote Blutkörperchen, Granulozyten und Lymphozyten, durch in Kontaktbringen der von Blut abgeleiteten Zusammensetzung nach dem Schritt des Waschens, der dem Schritt der Kultur vorausgeht, mit Antikörpern, die gegen alle oder ein Teil der vorstehend erwähnten Bestandteile gerichtet sind, und Wiedergewinnen der Lösung, die die Monozyten oder deren Vorstufen enthält, wobei alle oder ein Teil der vorstehend erwähnten Bestandteile auf den Antikörpern fixiert bleiben,
. und/oder gefolgt von einem Schritt der Eliminierung von allen oder einem Teil der Bestandteile, die sich von den Makrophagen oder MD-APC unterscheiden, durch Inkontaktbringen der von Blut abgeleiteten Zusammensetzung, die nach dem Schritt der Kultur erhalten wurde, mit den vorstehend beschriebenen Antikörpern und Wiedergewinnen der Lösung, die die Makrophagen oder MD-APC enthält, wobei alle oder ein Teil der vorstehend erwähnten Bestandteile an den Antikörpern fixiert bleibt,
. und/oder gefolgt von einem Schritt der Reinigung, insbesondere durch Elutriation, in welcher die Makrophagen oder die MD-APC, die gegebenenfalls aktiviert sind, von den anderen Bestandteilen der Zusammensetzung separiert werden, die nach dem vorstehend erwähnten Schritt der Kultur, und gegebenenfalls der Aktivierung erhalten wurde, insbesondere von Blutplättchen, roten Blutkörperchen und Lymphozyten auf physikalischem Weg, unter der Voraussetzung, dass es vor der Kultur der Zellen keinen Schritt der Separierung auf physikalischem Weg einerseits der Monozyten und andererseits der roten Blutkörperchen, der Granulozyten und des Großteils der Blutplättchen gibt.

2. Verfahren gemäß Anspruch 1, das eine Verdünnung der vom Blut abgeleiteten Zusammensetzung insbesondere in ungefähr 2 bis 3 Mal dem Volumen des letzteren mit Hilfe einer geeigneten physiologischen Lösung umfasst.

3. Verfahren gemäß Anspruch 1, **dadurch gekennzeichnet, dass** die von Blut menschlichen Ursprungs abgeleitete Ausgangszusammensetzung so ist, dass sie ein Teil der Monozyten oberhalb von ungefähr 5%, insbesondere von ungefähr 10% bis ungefähr 30%, bezogen auf die Zahl der weißen Blutkörperchen, die in der Zusammensetzung vorhanden sind, umfasst.

4. Verfahren gemäß Anspruch 1, **dadurch gekennzeichnet, dass** die von Blut menschlichen Ursprungs abgeleitete Ausgangszusammensetzung so ist, dass sie einen Anteil von roten Blutkörperchen umfasst, der weniger als ungefähr 3%, insbesondere 0,3% bis 0,5% beträgt, bezogen auf die Zahl der weißen Blutkörperchen, die in der Zusammensetzung vorhanden sind.

5. Verfahren gemäß einem der Ansprüche 1 bis 4, **dadurch gekennzeichnet, dass** die Ausgangszusammensetzung, die von menschlichen Blut abstammt, wie folgt ist:
- die Zahl der Plättchen umfasst ist zwischen ungefähr 2.10¹¹ bis ungefähr 6.10¹¹ auf 150ml bis 200ml,
- die Zahl der weißen Blutkörperchen umfasst ist zwischen ungefähr 5.10⁹ bis ungefähr 5.10¹⁰ auf 150ml bis 200ml,
- der Anteil der Lymphozyten umfasst ist zwischen ungefähr 60% bis ungefähr 80%, bezogen auf die Zahl der weißen Blutkörperchen, die in der Zusammensetzung vorhanden sind,
- der Anteil der Granulozyten umfasst ist zwischen ungefähr 10% bis ungefähr 20% bezogen auf die Zahl der weißen Blutkörperchen, die in der Zusammensetzung vorhanden sind.

6. Verfahren gemäß einem der Ansprüche 1 bis 5, **dadurch gekennzeichnet, dass** auf die Schritte der Kultur, Aktivierung und Zentrifugierung ein Schritt der Elutriation folgt und unter Verwendung der folgenden Vorrichtung realisiert wird:
- eine Tasche, die eine Zusammensetzung enthält, die die Makrophagen oder die MD-APC umfaßt, die gegenbenenfalls aktiviert sind, die nach dem Schritt der Kultur und gegebenenfalls der Aktivierung, erhalten wurde, genau so wie eine Tasche, die eine physiologische Lösung enthält (nachstehend als Elutriationslösung bezeichnet), die das ganze System der Elutriation in einem Milieu halten kann, das für die Makrophagen oder MD-APC, die gegebenenfalls aktiviert sind, geeignet ist, und ein Lufteinlass sind mit mit einem Filter ausgestatteten Transfusor mit 3 Armen verbunden,
- wobei der Lufteinlass geschlossen ist, eine peristaltische Pumpe den Inhalt der Tasche in einen Elutriator treibt, der mit einem Rotor mit einer Elutriationskammer ausgestattet ist, wobei die Einspeisung des Ganzen in den Elutriationskreislauf in die physiologische Flüssigkeit durch das Antreiben des Inhalts der Tasche, die die Elutriationslösung enthält, mit Hilfe der zuvor erwähnten Pumpe, während der ganzen Dauer der Elutriation sichergestellt wird,
- die Geschwindigkeit der Rotation des Rotors des Elutriators und der Umsatz der Pumpe sind so, dass die verschiedenen Bestandteile der Zusammensetzung der Tasche, die in den Rotor eingeführt werden, als Funktion ihrer Größe in die Elutrationskammer separiert werden, wobei die Bestandteile mit der kleinsten Größe als erstes über den Oberteil der Kammer (nahe dem Ausgang des Elutriators) wandern, so dass die Bestandteile mit der größten Größe, nämlich die Makrophagen, oder MD-APC, die gegebenenfalls aktiviert sind, als letztes zum Ausgang der Kammer wandern,
- die verschiedenen Bestandteile der Zusammensetzung, die in der Tasche enthalten sind, die so separiert wurden, werden in den mit dem Elutriator verbunden Taschen separat gesammelt, wobei die Blutplättchen und verschiedenen Rückstände mit kleiner Größe als erstes in einer ersten Tasche wiedergewonnen werden, die roten Blutkörperchen anschließend in einer zweiten Tasche wiedergewonnen werden, dann die Lymphozyten in den dritten und vierten Taschen wiedergewonnen werden, und schließlich die Makrophagen oder MD-APC in einer fünften Tasche wiedergewonnen werden, wobei die verschiedenen vorstehend erwähnten Kategorien der Bestandteile als Funktion ihrer jeweiligen Größe aus dem Elutriator extrahiert werden, sei es durch Vermindern der Geschwindigkeit der Rotation des Elutriators, sei es durch Erhöhen der Pumpleistung der Pumpe, wobei die Wiedergewinnung der Makrophagen oder MD-APC in der fünften Tasche vorzugsweise durch Anhalten des Rotors des Elutriators und Öffnen des Lufteinlasses realisiert wird, wobei die fünfte Tasche so die gereinigte Zusammensetzung von Makrophagen oder MD-APC, die gegebenenfalls aktiviert sind, vorzugsweise in einer im wesentlichen reinen Form enthält.

7. Verfahren gemäß einem der Ansprüche 1 bis 5, **dadurch gekennzeichnet, dass** der Schritt der Kultur, und gegebenenfalls der Aktivierung wie folgt ist:
- diesem ein Schritt der Kontaktherstellung der aus dem Schritt des Waschens der Ausgangszusammensetzung stammenden Zusammensetzung vorausgeht, die mit den Antiblutplättchen-, und/oder Antiroteblutkörperchen- und/oder Antigranulozytenantikörperchen, die auf einen insbesondere auf die nachstehend angegebene Weise angegebenen festen Träger fixiert sind,
obei das Inkontaktbringen der Zusammensetzung mit den Antikörpern sei es gleichzeitig mit den ganzen oder einen Teil der vorstehend erwähnten Antikörper, sei es aufeinanderfolgend insbesondere auf die vorstehend angegebene Weise, realisiert wird,
wobei die Zusammensetzung anschließend in die Tasche, die das Kulturmilieu enthält, transferiert wird,
- und gefolgt von einem Schritt des Inkontaktbringens der aus dem Schritt der Kultur und gegebenenfalls der Aktivierung stammenden Zusammensetzung mit den Antikörpern gegen Lymphozyten durch Einführen der Zusammensetzung in eine (oder mehrere) Tasche(n) oder andere Vorrichtung, wie vorstehend beschrieben, die die Antikörper gegen Lymphozyten enthält, und Wiedergewinnen der gereinigten Zusammensetzung aus Makrophagen oder MD-APC, die gegebenenfalls aktiviert sind, in im wesentlichen reiner Form.

8. Verwendung eines Kits zum Erhalt einer Zusammensetzung aus Makrophagen oder MD-APC, die gegebenenfalls aktiviert sind, gemäß einem der Ansprüche 1 bis 7, **dadurch gekennzeichnet, dass** dieser umfaßt:
. die Elemente, die für die Waschvorgänge, die Kultivierung und die Elutriation notwendig sind, wobei umfasst ist:
- eine (oder mehrere) Tasche(n) zum Waschen,
- eine (oder mehrere) vorzugsweise hydrophobe Tasche(n) zur Kultur,
- eine (oder mehrere) Verbindungsstück(e) zur Eliminierung des Überstands der Waschvorgänge,
- eine (oder mehrere) Verbindungsleitung(en) zwischen den vorhergehenden Taschen,
- gegebenenfalls eine (oder mehrere) Stelle(n) der Einspritzung in die Tasche(n) zum Waschen und zur Kultur,
- ein (oder mehrere) System(e), die die Öffnung oder den Verschluss von Verbindungen zwischen der (oder den) Tasche(n) zum Waschen und der (oder den) Tasche(n) zur Kultur erlauben, insbesondere Systeme mit Klammern auf den Leitungen,
- zwei Taschen, die die physiologische Lösung für die Waschvorgänge und die Elutriation enthalten,
- einen Transfusor mit drei Armen mit Lufteinleitung,
- eine Silikonleitung und ein Verbindungsstück zum Rotor, der zur Verbindung zwischen dem Transfusor und dem Rotor des Elutriators dient,
- eine (oder mehrere) Taschen zur Sammlung, die gegebenfalls durch Leitungen mit Klammern zusammengebunden sind,
- eine Silikonleitung und ein Verbindungsstück zum Rotor, der zur Verbindung zwischen der (oder den) Tasche(n) der Sammlung, die vorstehend erwähnt wurde(n), und dem Rotor des Elutriators dient,
die Reagenzien, die zur Kultivierung, Differenzierung, Aktivierung und Steuerung notwendig sind, die umfassen:
- eine Tasche mit spezifischem Kulturmilieu und fertig zur Verwendung, insbesondere ein Kulturmilieu auf der Basis von modifizierten IMDM, nämlich ein nach Iscove modifiziertes Dubelco-Medium (IMDM, Gibco), zu welchem L-Glutamin (2mM, Gibco) oder L-Alanyl-L-Glutamin (2mM, Gibco), Brenztraubensäure (2mM, Gibco), Indomethacine (5.10⁻⁶ M, Sigma), Mercaptoethanol (3.10⁻⁵ M, Gibco), nicht essentielle Aminosäuren (2%, Gibco); 2 bis 5% Serum AB⁺ (oder autologes Serum) hinzugegeben wird, genauso wie Antibiotika, wie etwa Penicillin und Streptomycin, im Moment der Kultur hinzugegeben werden,
- eines oder mehrere Zusatzstoffe, vorzugsweise in flüssiger oder gefriergetrockneter Form, zum Zugeben zum Kulturmilieu, ausgewählt aus den folgenden:
* GMCSF,
* Vitamin D3,
* Interleukin-13 (IL-13),
* IL-4,
* TNF-α
* Histamin,
- gegebenenfalls einen Aktivator, wie etwa Interferon-γ, vorzugsweise in flüssiger oder gefriergetrockneter Form,
- vorzugsweise eine interne Vergleichssubstanz oder mehrere interne Vergleichssubstanzen, die Monozyten und/oder Makrophagen oder MD-APC umfassen, wobei letztere gegebenenfalls aktiviert sind, vorzugsweise fixiert oder gefriergetrocknet, und gegebenenfalls markiert, oder kalibrierte Kugeln, gegebenenfalls markiert, insbesondere durch Fluoreszenz,
- eines oder mehrere insbesondere durch Fluoreszenz markierte Antikörper, wie etwa solche, die gegen die Antigene CD3 (Marker von Lymphozyten, die als negative Vergleichssubstanz verwendet werden), CD45 (Marker von Leukozyten im allgemeinen), CD14, CD16, CD64, CD54, CD58, CD80, CD86, HLA-DR und MAX-1 gerichtet sind.

9. Verwendung gemäß Anspruch 8, **dadurch gekennzeichnet, dass** der Kit sich in der Form von zwei Einheiten darstellt:
- wobei die Einheit I die Taschen, Leitungen, Stellen der Einspritzung, Klammersysteme, den Transfusor mit drei Armen, nämlich die sogenannten "trockenen" Elemente, enthält, die für den Waschvorgang, die Kultivierung und die Reinigung durch Elutriation verwendet werden,
- wobei die Einheit II eine Tasche enthält, die das spezifische Kulturmilieu genauso wie die Reagenzien zur Kultivierung, Differenzierung, Aktivierung und Steuerung der Qualität enthält.

10. Verwendung gemäß Anspruch 9, **dadurch gekennzeichnet, dass**:
. die Einheit I aus zwei Untereinheiten in der Form von zwei Gefäßen zusammengesetzt ist:
- wobei das Gefäß A das Material enthält, das für die Waschvorgänge der Zellen, die aus der Zytapherese stammen, notwendig ist, insbesondere:
* drei Taschen mit einer Kapazität von 600 ml (zentrale Tasche A1, und Empfangstaschen A2 und A3 in Fig. 1),
* ein Transferset zum Transferieren der Waschlösung in die Tasche (Tasche A1 in Fig. 1), in welcher die Blutzusammensetzung transferiert wird, um die aus der Zytapherese stammenden Zellen zu verdünnen und zu waschen,
* ein Set zum Transfer der gewaschenen Zellen (die in der Tasche A1 in der Fig. 1 enthalten sind) zu den Taschen der Kultur (Taschen B1, B2, und B3 in Fig. 2),
* ein Set zum Transfer des Kulturmilieus, zunächst zu den gewaschenen Zellen (die in der Tasche A1 in Fig. 1 enthalten sind), und als zweites zu den vorstehend erwähnten Taschen der Kultur,
* drei hydrophobe Taschen der Kultur mit einer Kapazität von 3 Litern (Taschen B1, B2 und B3 in Fig. 2),
- wobei das Gefäß B die Elemente zum Separieren der Makrophagen oder differenzierten MD-APC von den anderen Zellen, die in der Kultur bei dem Schritt der Elutriation vorhanden sind, enthält, insbesondere:
* eine Tasche zum Zusammenführen (oder Tasche zum Poolen B4 in Fig. 2),
* einen Transfusor mit drei Armen mit einer Lufteinleitung,
* eine Silikonleitung, und zwei Verbindungsstücke zum Rotor, wobei die Leitung und eines der Verbindungsstücke zur Verbindung zwischen dem Transfusor und dem Rotor des Elutriators dient, das andere Verbindungsstück zur Verbindung mit dem Rotor und der nachstehenden Einheit von Taschen dient, wobei die letztere Leitung eine Stelle zur Probeentnahme enthält,
* eine Einheit, die aus vier Sammeltaschen (Taschen C1, C2, C3 und C4 in Fig. 3) zusammengesetzt ist, mit einer Kapazität von 1 Liter, aus einer Tasche (Tasche E1, in der Fig. 3) mit einer Kapazität von 600 ml, um die Endfraktion der Makrophagen oder gereinigten MD-APC zu enthalten, aus einer Tasche (Tasche E2 in Fig. 3) mit einer Kapazität von 600 ml zum Wiedergewinnen des Überstands der vorausgehenden Tasche, und aus einer Tasche (Tasche E3 in Fig. 3) zum Einspritzen von Makrophagen oder MD-APC, die gegebenenfalls aktiviert sind, in Patienten,
. die Einheit II durch das Gefäß C dargestellt wird, wobei das letztere eine Thermostasche ist, gegebenenfalls mit Kühler, um die Temperatur von 4°C zu halten, und die insbesondere enthält:
- eine Tasche mit zwei Litern Kulturmilieu auf der Basis von IMDM, das wie vorstehend beschrieben modifiziert ist,
- ein Gefäß, das folgende flüssige oder gefriergetrocknete Produkte enthält:
* einen Kolben mit Antibiotika (Penicillin, vorzugsweise 1000 U/Streptomycin, vorzugsweise 1000 µg),
* einen Kolben mit Vitamin D3 (vorzugsweise 4 bis 8 µg),
* einen Kolben mit GMCSF (vorzugsweise 5.10⁵ bis 10⁶ U),
* einen Kolben mit Interferon γ (vorzugsweise 25.10⁴ bis 5.10⁵ U)
* einen Kolben mit IL-13,
* einen Kolben mit IL-4,
* einen Kolben mit TNFα,
* einen Kolben mit Histamin,
- ein Gefäß, das eine interne Vergleichsubstanz oder mehrere interne Vergleichssubstanzen, wie vorstehend beschrieben, enthält, und ein oder mehrere Kolben, die Antikörper, Anti-CD3, Anti-CD45, Anti-CD14, Anti-CD16, Anti-CD64, Anti-MAX-1, Anti-CD54, Anti-CD58, Anti-CD80, Anti-CD86 und Anti-HLA-DR, die insbesondere durch Kupplung mit Fluorescin, markiert sind.

11. Kit zum Erhalt von Zusammensetzungen von Makrophagen oder von MD-APC, die gegebenenfalls aktiviert sind, **dadurch gekennzeichnet, dass** dieses umfasst:
- eine (oder mehrere) Tasche(n), wobei im Inneren von dieser Antikörper gegen rote Blutkörperchen fixiert sind,
- eine (oder mehrere) Tasche(n), wobei im Inneren von dieser (diesen) Antikörper gegen Blutplättchen fixiert sind,
- eine (oder mehrere) Tasche(n), wobei im Inneren von dieser (diesen) Antikörper gegen Granulozyten fixiert sind,
- eine (oder mehrere) Tasche(n), wobei im Inneren von dieser (diesen) Antikörper gegen Lymphozyten fixiert sind,
- eine (oder mehrere) Tasche(n) der Kultur,
- eine (oder mehrere) Tasche(n) der Wiedergewinnung von Makrophagen oder MD-APC, die gegebenenfalls aktiviert und gereinigt sind,
- Leitungen von Verbindungsstück zwischen den verschiedenen zuvor erwähnten Taschen,
- Zusatzstoffe, vorzugsweise in flüssiger oder gefilterter trockener Form, zum Zugeben zum Kulturmilieu, insbesondere:
* Antibiotika, wie etwa Penicillin und Streptomycin,
* GMCSF,
* Vitamin D3,
* IL-13,
* IL-4,
* TNF-α
* Histamin,
- gegebenenfalls einen Aktivator, wie etwa Interferon-γ, vorzugsweise in flüssiger oder gefriergetrockneter Form,
- vorzugsweise eine interne Vergleichssubstanz (oder mehrere interne Vergleichssubstanzen), die Monozyten oder Makrophagen oder MD-APC, die gegebenenfalls aktiviert sind, umfassen, die vorzugsweise fixiert sind, und gegebenenfalls markiert, oder kalibrierte Kugeln, die gegebenenfalls markiert sind, insbesondere durch Fluoreszenz, wie etwa vorstehend beschrieben,
- markierte Antikörper, insbesondere durch Fluoreszenz, wie etwa diejenigen, die gegen Antigene CD3, CD45, CD14, CD16, CD64; CD54, CD58, CD80, CD86, MAX-1 und HLA-DR gerichtet sind.

12. Verwendung eines Kits gemäß Anspruch 11, um ein Verfahren gemäß den Ansprüchen 1 bis 5 und 7 in Gang zu setzen.

## Claims

1. A process for preparing a composition comprising macrophages, in particular activated macrophages, or macrophages, or other cells derived from monocytes, or precursors of the latter, presenting at their surface specific antigens which are capable of inducing a specific immune response (also designated MD-APCs), said MD-APCs being possibly activated, **characterized in that**, starting from a composition derived from blood of human origin, rich in blood cells, and more specifically in white blood cells such as monocytes, or in precursors of the latter, in particular a composition derived from blood such as that obtained by cytapheresis, said procedure including the following stages:
- a stage of washing said composition derived from blood, advantageously by simple centrifugation, and washing the pellet issued from said centrifugation, after recovery of the pellet in an appropriate physiologic washing solution, in particular in a bag (of transfer type), by effecting for example a pressure on said bag, the washing solution thus being eliminated to another bag or other recipient, to recover a composition devoid of possible anticoagulants and various residues and impoverished in platelets,
- a stage of culture of the cells contained in the composition derived from the blood obtained after the stage of washing mentioned above, by placing it in an appropriate culture medium, in particular in a bag, advantageously hydrophobic, for about 6 to about 10 days (in particular from about 6 to 7 days), this stage of culture possibly taking place in the presence of the above mentioned specific antigens, namely in particular in the presence of antigens characteristic of tumor cells or of pathogens in the case of production of MD-APCs, and possibly followed by a stage of activation of the macrophages or MD-APCs obtained in the culture medium, by adding an activator in said culture medium, such as interferon γ, said activator being placed in contact with the contents of the culture medium for about 16 to about 24 hours, said stage of culture, and if necessary of activation, being possibly followed by a centrifugation of the culture medium and a wash of the pellet issued from the centrifugation, in particular in the manner indicated above,
said stage of culture, and if necessary of activation, being:
• preceded by a stage of elimination of all or part of the constituents other than the monocytes or their precursors, able to be present in the starting composition, in particular the platelets, red blood cells, granulocytes, and lymphocytes, by contacting the composition derived from blood obtained after the washing stage preceding the culture stage, with antibodies directed against all or part of the above mentioned constituents, and recovery of the solution containing the monocytes, or their precursors, all or part of the above mentioned constituents remaining fixed to the antibodies,
• and/or followed by a stage of elimination of all or part of the constituents other than the macrophages or MD-APCs by being placed in contact with the composition derived from blood obtained after the culture stage with the antibodies such as described above, and recovery of the solution containing the macrophages or MD-APCs, all or part of the above mentioned constituents remaining fixed to the antibodies,
• and/or followed by a purification stage, in particular an elutriation stage in which the macrophages or MD-APC possibly activated are separated from the other constituents of the composition obtained after the stage of culture, and possibly the stage of activation mentioned above, in particular the platelets, red blood cells, and lymphocytes, by physical means, under the proviso that before the cell culture there is no separation stage by physical means, of the monocytes on the one hand, and of the red blood cells, granulocytes and most of the platelets on the other hand.

2. A process according to claim 1, comprising a dilution of said composition derived from blood, in particular in about 2 to 3 times the volume of the latter, by means of a suitable physiological solution.

3. A process according to claim 1 or claim 2, **characterized in that** the starting composition derived from blood of human origin is such that it includes a proportion of monocytes greater than about 5%, in particular of about 10% to about 30%, in number of white blood cells present in said composition.

4. A process according to claim 1, **characterized in that** the starting composition derived from blood of human origin is such that it includes a proportion of red blood cells less than about 3%, in particular from 0.3% to 0.5%, in number of white blood cells present in the said composition.

5. A process according to one of the claims 1 to 4, **characterized in that** the starting composition derived from blood of human origin is such that:
- the number of platelets is comprised between about 2 × 10¹¹ to about 6 × 10¹¹ in 150 ml to 200 ml,
- the number of white blood cells is comprised between about 5 × 10⁹ to about 5 × 10¹⁰ in 150 ml to 200 ml,
- the proportion of lymphocytes is comprised between about 60% to about 80%, in number of white blood cells present in said composition,
- the proportion of granulocytes is comprised between about 10% to about 20% in number of white blood cells present in said composition.

6. A process according to one of claims 1 to 5, **characterized in that** the stages of culture, activation and centrifugation are followed by a stage of elutriation, and carried out by using the following mechanism:
- a bag containing the composition comprising macrophages or MD-APCs, possibly activated, obtained after the stage of culture, and if necessary of activation, as well as a bag containing a physiologic solution (also designated by elutriation solution) able to maintain the whole elutriation system in a medium viable for the macrophages or MD-APCs, possibly activated, and an air intake, are linked to a transfuser with three branches equipped with a filter,
- once the air intake is closed, a peristaltic pump pulls the contents of the bag into an elutriator equipped with a rotor with an elutriation chamber, the feeding of the entirety of the elutriation circuit with physiologic liquid being assured by pulling of the contents of the bag containing the elutriation solution, with the above mentioned pump, during the entire duration of the elutriation,
- the speed of rotation of the elutriation rotor and the delivery from the pump are such that the various constituents of the composition of bag, introduced into the rotor, are separated according to their size into the elutriation chamber, the constituents of the smallest size migrating firstly to the top of the chamber (near the exit of the elutriator), while the constituents of the largest size, namely the macrophages or MD-APCs, possibly activated, migrating to the exit of the chamber lastly,
- the different constituents of the composition in bag thus separated are harvested separately into bags linked to the elutriator, the platelets and various small size residues being recovered first into a first bag, the red blood cells being then recovered into a second bag, the lymphocytes into the third and fourth bags, and lastly the macrophages or MD-APCs into a fifth bag, the different categories of constituents mentioned above thus being extracted from the elutriator according to their respective size, either by diminishing the speed of the rotation of the elutriator, or by increasing the delivery of the pump, the recovery of macrophages or MD-APCs into the fifth bag advantageously being able to be carried out by stopping the rotor of the elutriator and opening the air intake, the fifth bag thus containing the composition of macrophages or MD-APCs, possibly activated, being sought, advantageously in an essentially pure form.

7. A process according to claims 1 to 5, **characterized in that** the stage of culture, and if necessary of activation, is:
- preceded by a stage of contacting the composition issued from the stage of washing the starting composition, with the anti-platelet and/or anti-red blood cell and/or anti-granulocyte antibodies fixed on a solid support, in particular in the way indicated above,
the contact of said composition with said antibodies being carried out either simultaneously with all or part of the above mentioned antibodies, or successively in particular in the manner indicated above,
said composition being then transferred into the bag containing the culture medium,
- and followed by a stage of contacting the composition issued from the stage of culture, and possibly of activation, with the anti-lymphocyte antibodies, by introduction of said composition into one (or several) bag(s) or another device such as described above, containing anti-lymphocyte antibodies, and recovery of the composition being sought of macrophages or MD-APCs, possibly activated, in an essentially pure form.

8. Use of a kit (or case) for obtaining compositions of macrophages or MD-APCs, possibly activated, according to one of claims 1 to 7, **characterized in that** it includes:
• the necessary elements for the washings, the culture and the elutriation, including:
- one (or several) bag(s) for washing,
- one (or several) culture bag(s), preferably hydrophobic,
- one (or several) connector(s) for eliminating the supernatant from the washings,
- one (or several) connecting tube(s) between the preceding bags,
- possibly, one (or several) injection site(s) in the washing and culture bag(s),
- one (or several) system(s) allowing for the opening or closing of the links between the bag(s) for washing and the culture bag(s), in particular systems of clamps on the tubes,
- two bags containing the physiologic solution for the washings and the elutriation,
- a three branch transfuser with air intake,
- a silicon tube and a joint on the rotor serving as a connection between the transfuser and the rotor of the elutriator,
- one (or several) collection bag(s), possibly linked amongst themselves by tubes equipped with a clamp system,
- a tube and a joint on the rotor serving as a connection between the collection bag(s) mentioned above and the rotor of the elutriator,
• the necessary reactive agents for the culture, differentiation, activation and control, including:
- a bag of specific and ready-to-use culture medium, in particular a culture medium constituted from modified IMDM, namely Iscove modified Dubelcco medium (IMDM, Gibco), to which has been added L-Glutamine (2 mM, Gibco) or L-Alanyl-L-Glutamine (2 mM, Gibco), pyruvic acid (2 mM, Gibco), Indomethacine (5 × 10⁻⁶ M, Sigma), mercaptoethanol (3 × 10⁻⁵ M, Gibco), non-essential amino acids (2%, Gibco); 2 to 5% of AB⁺ serum (or autologous serum), as well as antibiotics such as penicillin and streptomycin, added at the moment of culture,
- one or several supplements advantageously under liquid or lyophilized form, to be added to the culture medium, chosen from among the following:
* GMSCF,
* vitamin D3,
* interleukin-13 (IL-13)
* IL-4
* TNF-α
* histamine
- possibly, an activator such as interferon γ, advantageously under liquid or lyophilized form,
- advantageously one (or several) internal control(s) comprising monocytes, and/or macrophages or MD-APCs, the latter being possibly activated, advantageously fixed, or lyophilized, and possibly labelled, or calibrated beads, possibly labelled, in particular by fluorescence,
- one or several labelled antibodies, in particular by fluorescence, such as those directed against antigens CD3 (marker for lymphocytes used in case of negative control), CD45 (marker for leukocytes in general), CD14, CD16, CD64, CD54, CD58, CD80, CD86, HLA-DR and MAX-1.

9. Use according to claim 8, **characterized in that** the kit is presented in two sets:
- set I containing the bags, tubes, injection sites, clamp systems, three branch transfuser, namely the "dry" elements used for the washings, the culture and the purification by elutriation,
- set II containing a bag containing the specific culture medium as well as the reactive agents for the culture, differentiation, activation and quality control.

10. Use according to claim 9, **characterized in that**:
• set I is consisted of two sub-sets in the form of two boxes:
- box A containing the material necessary for washing the cells obtained from the cytapheresis, in particular:
* three bags with a capacity of 600 ml (central bag A1, and recovery bags A2 and A3 shown in figure 1),
* a transfer set for transferring the washing solution into the bag (bag A1 in figure 1) in which the blood composition is transferred in order to dilute and wash the cells issued from cytapheresis,
* a transfer set for the washed cells (contained in bag A1 in figure 1) to the culture bags (bags B1, B2, and B3 in figure 2),
* a transfer set for the culture medium, firstly towards the washed cells (contained in bag A1 in figure 1), and secondly towards the culture bags mentioned above,
* three hydrophobic culture bags with a capacity of 3 litres (bags B1, B2, and B3 in figure 2),
- box B containing the elements for the separation of the differentiated macrophages or MD-APCs from the other cells present in the culture during the stage of elutriation, in particular:
* a gathering bag (or pooling bag B4 in figure 2),
* a three branch transfuser with air intake,
* a silicon tube, and two joints to the rotor, the tube and one of the two joints serving as a connection between the transfuser and the rotor of the elutriator, the other joint serving as a connection with said rotor and the group of bags mentioned hereafter, the latter tube containing a site for sample withdrawal,
* a combination of four recovery bags (bags C1, C2, C3, and C4 in figure 3) with a capacity of one litre, a bag (bag E1 in figure 3) with a capacity of 600 ml to contain the final fraction of purified macrophages or MD-APCs, and a bag (bag E2 in figure 3) with a capacity of 600 ml for the recovery of the supernatant of the preceding bag, and a bag (bag E3 in figure 3) for injecting the patients with macrophages or MD-APCs, possibly activated,
• set II is represented by box C, the latter being an isothermal box, possibly with a cooling system to maintain the temperature at 4°C, and containing in particular:
- a bag of 2 litres of culture medium primarily constituted from modified IMDM, as described above,
- a box containing the following liquid or lyophilized products:
* a flask of antibiotics (penicillin, advantageously 1,000 U/streptomycin, advantageously 1,000 µg),
* a flask of vitamin D3 (advantageously 4 to 8 µg),
* a flask of GMSCF (advantageously 5 × 10⁵ to 10⁶ U),
* a flask of interferon γ (advantageously 25 × 10⁴ to 5 × 10⁵ U),
* a flask of IL-13,
* a flask of IL-4,
* a flask of TNFα,
* a flask of histamine,
- a box containing one (or several) internal control(s) as described above, and one or many flasks containing the labelled antibodies anti-CD3, anti-CD45, anti-CD14, anti-CD16, anti-CD64, anti-MAX-1, anti-CD54, anti-CD58, anti-CD80, anti-CD86, and anti-HLA-DR, in particular labelled with fluoresceine.

11. A kit for obtaining compositions of macrophages or MD-APCs, possibly activated, **characterized in that** it includes:
- one (or several) bag(s) inside of which are found fixed anti-red blood cell antibodies,
- one (or several) bag(s) inside of which are found fixed anti-platelet antibodies,
- one (or several) bag(s) inside of which are found fixed anti-granulocyte antibodies,
- one (or several) bag(s) inside of which are found fixed anti-lymphocyte antibodies,
- one (or several) culture bag(s),
- one (or several) bag(s) for the recovery of purified macrophages or MD-APCs, possibly activated,
- tubes joining the purified different bags mentioned above,
- supplements advantageously under liquid or lyophilized form, to be added to the culture medium, in particular:
* antibiotics such as penicillin and streptomycin,
* GMCSF,
* vitamin D3
* IL-13,
* IL-4,
* TNFα,
* histamine,
- possibly, an activator such as interferon γ, advantageously under liquid or lyophilized form,
- advantageously one (or several) internal control(s) comprising monocytes or macrophages or MD-APCs, possibly activated, advantageously fixed, and possibly labelled, or calibrated beads, possibly labelled, in particular by fluorescence, as described above,
- labelled antibodies, in particular by fluorescence, such as those directed against the antigens CD3, CD45, CD14, CD16, CD64, CD54, CD58, CD80, CD86, MAX-1, and HLA-DR.

12. The use of a kit according to claim 11 for carrying out a process according to anyone of claims 1 to 5 and 7.
